(19) Europäisches Patentamt
European Patent Office
Office européen des brevets

(11) EP 3 101 025 A1

(12) **EUROPEAN PATENT APPLICATION**
published in accordance with Art. 153(4) EPC

(43) Date of publication:
**07.12.2016 Bulletin 2016/49**

(21) Application number: **15743891.2**

(22) Date of filing: **28.01.2015**

(51) Int Cl.:
***C07J 9/00*** *(2006.01)*

(86) International application number:
**PCT/KR2015/000910**

(87) International publication number:
**WO 2015/115796 (06.08.2015 Gazette 2015/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **29.01.2014 KR 20140011339**

(71) Applicant: **Humedix Co. Ltd.**
**Seongnam-si, Gyeonggi-do 463-400 (KR)**

(72) Inventors:
- **CHUNG, Bong Youl**
  **Yongin-si**
  **Gyeonggi-do 448-836 (KR)**
- **BANG, Sung Sik**
  **Hwaseong-si**
  **Gyeonggi-do 445-737 (KR)**
- **YOO, Min Ji**
  **Bucheon-si**
  **Gyeonggi-do 420-761 (KR)**
- **KIM, Myung Su**
  **Nonsan-si**
  **Chungcheongnam-do 320-800 (KR)**
- **JEONG, Min Wook**
  **Suwon-si**
  **Gyeonggi-do 441-768 (KR)**
- **JEONG, In Wha**
  **Wonju-si**
  **Gangwon-do 220-756 (KR)**
- **KIM, Yong Soo**
  **Ansan-si**
  **Gyeonggi-do 426-742 (KR)**

(74) Representative: **V.O.**
**P.O. Box 87930**
**Carnegieplein 5**
**2508 DH Den Haag (NL)**

(54) **PEGYLATED 7-DEHYDROCHOLESTEROL DERIVATIVE**

(57) The present invention relates to pegylated 7-dehydrocholesterol derivatives, and a composition for wrinkle alleviation and anti-aging comprising the same. The pegylated 7-dehydrocholesterol derivatives according to the present invention have improved stability and solubility in water, and thus can be effectively used as a good source for supplying vitamin D in a cosmetic composition, a pharmaceutical composition and a functional food for wrinkle alleviation and anti-aging.

【FIGURE 1】

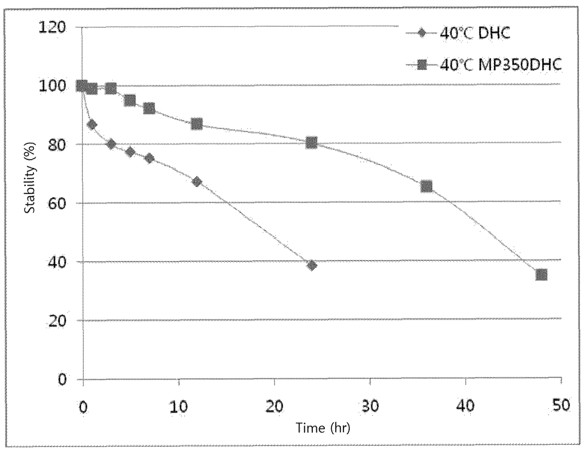


EP 3 101 025 A1

## Description

[Technical Field]

The present invention relates to pegylated 7-dehydrocholesterol derivatives. More particularly, the present invention relates to pegylated 7-dehydrocholesterol derivatives which have improved stability and solubility and are hydrolyzed *in vivo* to supply vitamin D, and a composition for wrinkle alleviation and anti-aging comprising the same.

[Background Art]

Vitamin D has two types of vitamin $D_2$ (ergocalciferol) and vitamin $D_3$ (cholecalciferol, Compound B), and these two substances are subject to metabolism in liver and kidney to be converted into their active form (calcitriol, Compound C). 7-dehydrocholesterol (7-DHC, Compound A) is a provitamin form of vitamin $D_3$ and thus is converted into vitamin $D_3$ by sunlight.

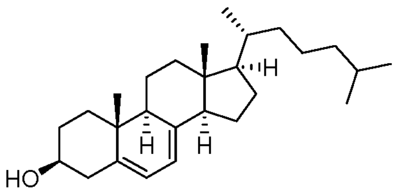

A

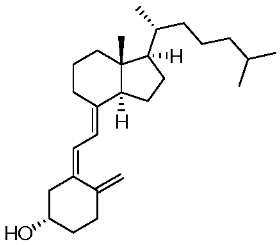

B

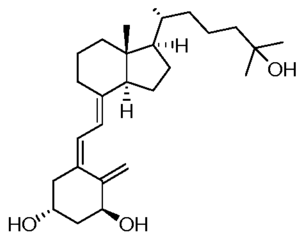

C

Vitamin $D_3$ generally promotes calcium absorption to strengthen the density of bone, so it is used as a therapeutic agent of osteoporosis and as a supplement for elderly or residents in northern latitudes where can be hard to get enough sunshine.

The 7-dehydrocholesterol known as a precursor of vitamin D has potent anti-aging and anti-oxidative activities to skin and the effect of protecting skin from ultraviolet rays, and thus is used as a raw material of functional cosmetics [*see:* Korean Patent Application Publication No. 2001-0002281]. Also, US Patent Application Publication No. 2013-0017234 discloses that 7-dehydrocholesterol has a possibility of being used for arthritis treatment as it makes factors of Col-1, ALP, OSX, OC, BMP-2 and IL-5 which are involved in the formation of bone be expressed. In addition, Korean Patent No. 10-0568600 discloses that 7-dehydrocholesterol has the effect of excellent hair protection through clinical tests.

Accordingly, it is expected that the continuous supplement of 7-dehydrocholesterol in the outer layer of skin can increase the production of cholecalciferol to prevent osteoporosis and prevent psoriasis characterized by patches of abnormal skin and skin diseases such as an atopic dermatitis, as well as providing the effect of blocking ultraviolet absorption and anti-aging.

However, 7-dehydrocholesterol is very unstable in regions other than skin and is converted into several substances by light, and those substances are very difficult to be converted into vitamin $D_3$ although they have little side effects. Also, 7-dehydrocholesterol is converted into pyrocalciferol or lumisterol and isopyrocalciferol to exhibit reduced activity in regions other than skin.

In order to overcome these problems, US Patent No. 5,342,833 discloses the use of a novel cholecalciferol having a new property, i.e. an active vitamin D derivative, in treatment of skin diseases and psoriasis. Also, US Patent No. 5,747,478 discloses the stabilization of activated cholecalciferol by using magnesium oxide and sodium citrate.

However, the studies for stabilizing 7-dehydrocholesterol is very marginal because 7-dehydrocholesterol is apt to be converted into other substances by ultraviolet rays and temperature and it has the structural problem of being denatured by reaction with oxygen in air.

Also, as mentioned above, the prior patents merely disclose the pharmaceutical stabilization of 7-dehydrocholesterol or show the studies on cholecalciferol as an active form. Thus, there is no disclosure on synthetic stabilization of 7-dehydrocholesterol.

[Disclosure]

[Technical Problem]

The present inventors have researched to overcome the above problems of 7-dehydrocholesterol being unstable to chemicals, ultraviolet, temperature and contact with air and having poor solubility in water, and found that the conjugation of 7-dehydrocholesterol with polyethylene glycol can increase solubility in an organic solvent or an aqueous solution and can raise the effect of transferring it to cells within skin, and the improvement of its properties can promote the improvement of efficacy, thereby enhancing the solubility and stability for a long-term storage of 7-dehydrocholesterol.

An object of the present invention is, therefore, to provide pegylated 7-dehydrocholesterol derivatives having improved solubility and stability.

Another object of the present invention is to provide a composition for wrinkle alleviation and anti-aging, comprising the pegylated 7-dehydrocholesterol derivatives.

[Technical Solution]

The present invention relates to a pegylated 7-dehydrocholesterol derivative of the following formula (I):

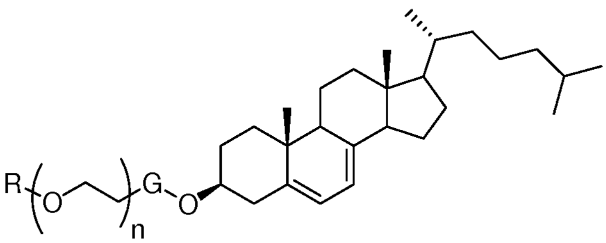

(I)

wherein,

R is hydrogen or $C_1$-$C_6$ alkyl,
G is $-XCOCH_2(CH_2)_mCO-$, $-XCH_2(CH_2)_mCO-$, -XCONHCYCO-,$-XCOCYNHCO(CH_2)_mCH_2CO-$ or $-XCH_2(CH_2)_mCONHCYCO-$,
X is O, NH or S,
Y is hydrogen, $C_1$-$C_6$ alkyl, $(CH2)_mCH2NHCO(CH_2)_mCH_2(OCH_2CH_2)_nOR$, $(CH_2)_{m'}CH_2NHCOCH_2(CH_2)_mCO(OCH_2CH_2)_nOR$ or $(CH_2)_{m'}CH_2NHCOCH_2(CH_2)_mCO$-DHC,
n is an integer of 1 to 700, preferably an integer of 3 to 200, and
m and m' are each independently an integer of 1 to 3, preferably 1.

The term "$C_1$-$C_6$ alkyl" as used herein means a straight or branched hydrocarbon having 1 to 6 carbon atoms, which includes methyl, ethyl, n-propyl, i-propyl, n-butyl, i-butyl, s-butyl, t-butyl, n-pentyl, n-hexyl and the like, but is not limited thereto.

In one embodiment of the present invention, the pegylated 7-dehydrocholesterol derivative may be a compound of the following formula (1A):

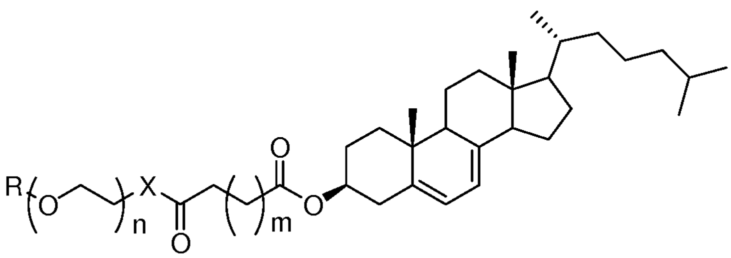

(1A)

wherein,

R is hydrogen or $C_1$-$C_6$ alkyl,

X is O or NH,
n is an integer of 1 to 700, preferably an integer of 3 to 200, and
m is an integer of 1 to 3, preferably 1.

In one embodiment of the present invention, the pegylated 7-dehydrocholesterol derivative may be a compound of the following formula (1B):

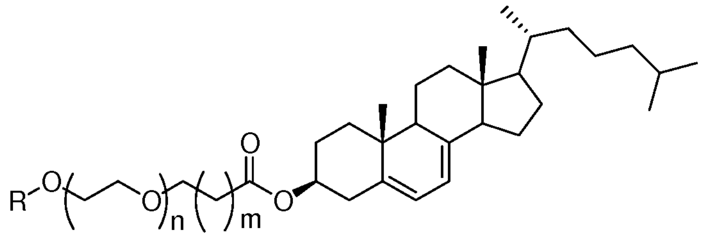

(1B)

wherein,

R is hydrogen or $C_1$-$C_6$ alkyl,
n is an integer of 1 to 700, preferably an integer of 3 to 200, and
m is an integer of 1 to 3, preferably 1.

In one embodiment of the present invention, the pegylated 7-dehydrocholesterol derivative may be a compound of the following formula (2A):

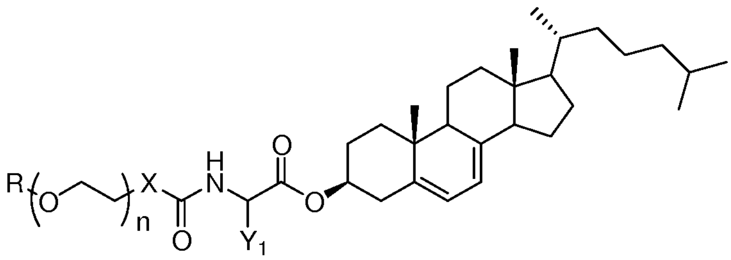

(2A)

wherein,

R is hydrogen or $C_1$-$C_6$ alkyl,
X is O or NH,
n is an integer of 1 to 700, preferably an integer of 3 to 200, and
$Y_1$ is $C_1$-$C_6$ alkyl.

In one embodiment of the present invention, the pegylated 7-dehydrocholesterol derivative may be a compound of the following formula (2B):

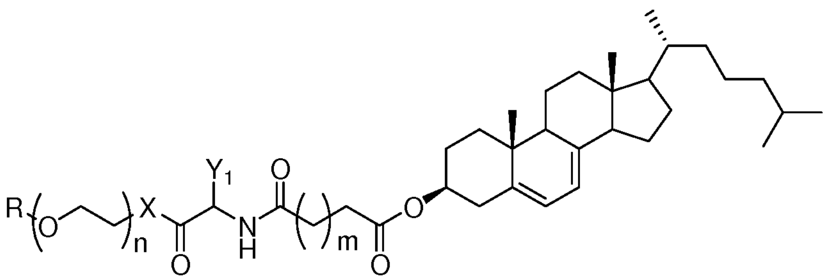

(2B)

wherein,

R is hydrogen or $C_1$-$C_6$ alkyl,
X is O or NH,
n is an integer of 1 to 700, preferably an integer of 3 to 200,
m is an integer of 1 to 3, preferably 1, and
$Y_1$ is $C_1$-$C_6$ alkyl.

In one embodiment of the present invention, the pegylated 7-dehydrocholesterol derivative may be a compound of the following formula (2C):

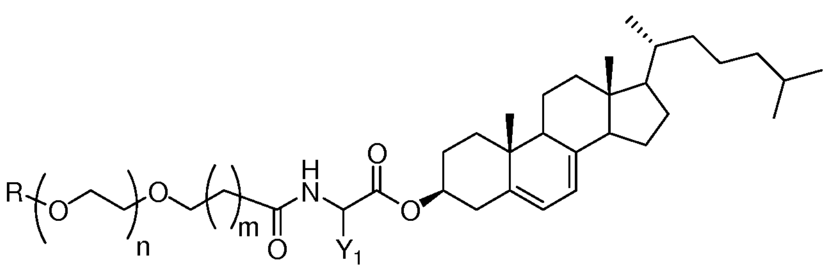

(2C)

wherein,

R is hydrogen or $C_1$-$C_6$ alkyl,
n is an integer of 1 to 700, preferably an integer of 3 to 200,
m is an integer of 1 to 3, preferably 1, and
$Y_1$ is $C_1$-$C_6$ alkyl.

In one embodiment of the present invention, the pegylated 7-dehydrocholesterol derivative may be a compound of the following formula (2D):

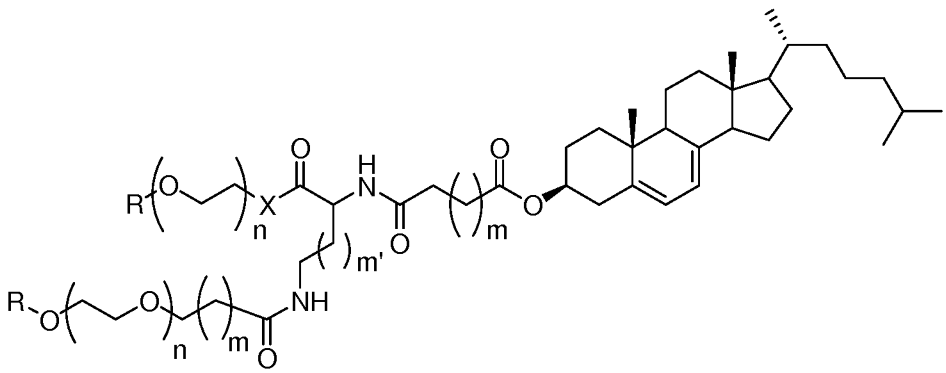

(2D)

wherein,

R is hydrogen or $C_1$-$C_6$ alkyl,
X is O or NH,
n is an integer of 1 to 700, preferably an integer of 3 to 200, and
m and m' are each independently an integer of 1 to 3, preferably 1.

In one embodiment of the present invention, the pegylated 7-dehydrocholesterol derivative may be a compound of the following formula (2E):

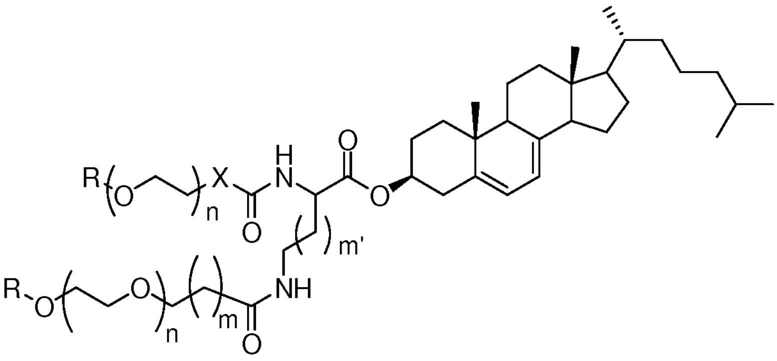

(2E)

wherein,

R is hydrogen or $C_1$-$C_6$ alkyl,
X is O or NH,
n is an integer of 1 to 700, preferably an integer of 3 to 200, and
m and m' are each independently an integer of 1 to 3, preferably 1.

In one embodiment of the present invention, the pegylated 7-dehydrocholesterol derivative may be a compound of the following formula (2F):

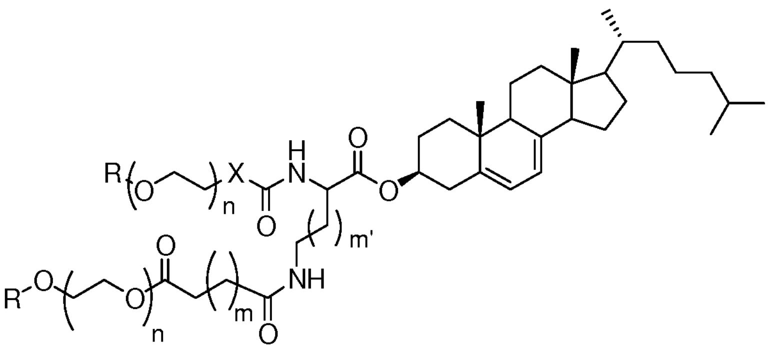

(2F)

wherein,

R is hydrogen or $C_1$-$C_6$ alkyl,
X is O or NH,
n is an integer of 1 to 700, preferably an integer of 3 to 200, and
m and m' are each independently an integer of 1 to 3, preferably 1.

In one embodiment of the present invention, the pegylated 7-dehydrocholesterol derivative may be a compound of the following formula (2G):

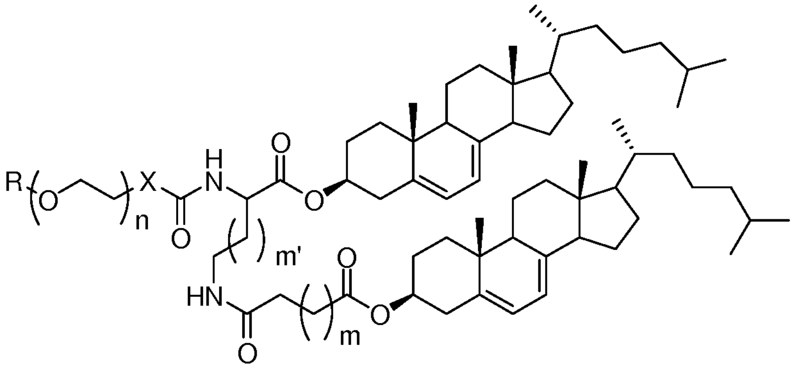

(2G)

wherein,

R is hydrogen or $C_1$-$C_6$ alkyl,
X is O or NH,
n is an integer of 1 to 700, preferably an integer of 3 to 200, and
m and m' are each independently an integer of 1 to 3, preferably 1.

The pegylated 7-dehydrocholesterol derivatives according to the present invention may be prepared by methods shown in the following Reaction Schemes 1 to 5. The methods of Reaction Schemes merely illustrate those being representatively used in the present invention, and the sequence of unit operations, reagents and reaction conditions in the Reaction Schemes may be properly modified.

[Reaction Scheme 1]

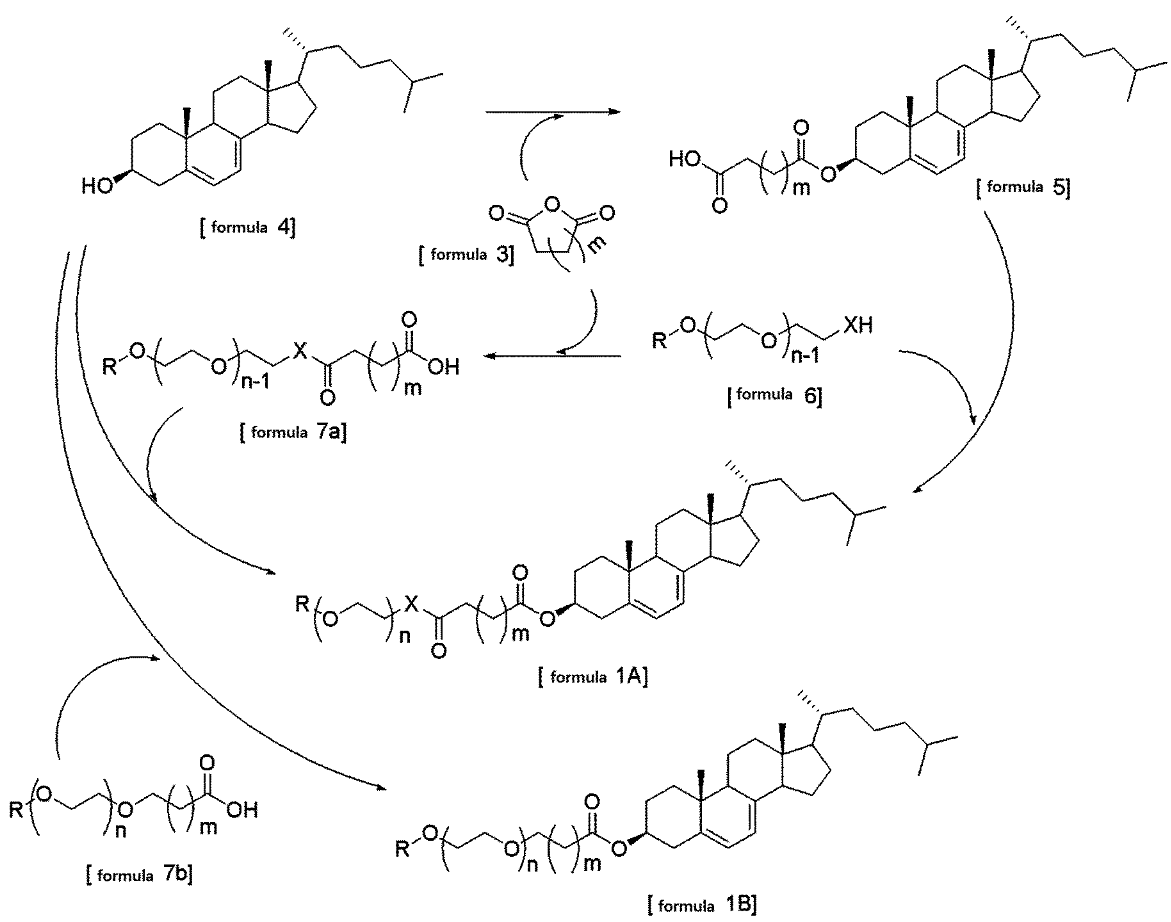

[formula 4]
[formula 3]
[formula 5]
[formula 6]
[formula 7a]
[formula 1A]
[formula 7b]
[formula 1B]

wherein,
R, X, n and m are the same as defined in the above formula (I).

According to Reaction Scheme 1, the compound of formula (4) and the compound of formula (5) may be subject to condensation reaction with the compound of formula (7a) or the compound of formula (6), respectively, to obtain a pegylated 7-dehydrocholesterol derivative of formula (1A). Also, the compound of formula (4) may be subject to condensation reaction with the compound of formula (7b) to obtain a pegylated 7-dehydrocholesterol derivative of formula (1B).

The compound of formula (5) and the compound of formula (7a) may each be prepared by reaction of the compound of formula (4) or the compound of formula (6) with an acid anhydride, and the compound of formula (7b) may be prepared by using the compound of formula (6) wherein X is O as a starting material according to the known method or may be commercially available [*see:* J.M. Harris et.al, Advanced Drug Delivery Reviews, 2002, 54, 459-476].

Also, the compound of formula (6) may be commercially available or may be prepared by the known method

[*see:* Sandler and Karo, Polymer Synthesis, Academic Press, New York, Vol.3, pages 138-161].

[Reaction Scheme 2]

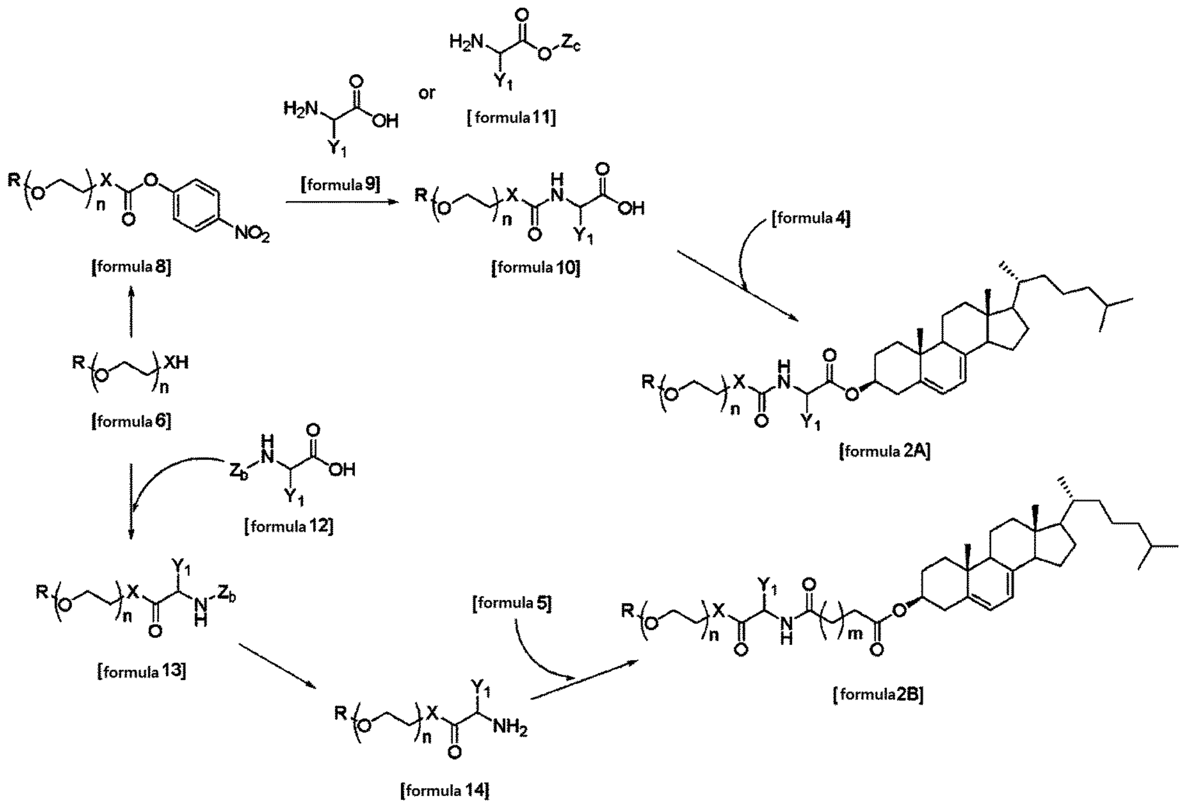


wherein,

R, X, n and m are the same as defined in the above formula (I),
$Z_b$ and $Z_c$ are each a protecting group, and
$Y_1$ is hydrogen, $C_1$-$C_6$ alkyl or an amino acid residue.

According to Reaction Scheme 2, the compound of formula (6) may be activated to obtain the compound of formula (8), which is subject to reaction with the compound of formula (9) to obtain a polyethylene glycol derivative of formula (10). Also, the compound of formula (10) may be subject to condensation reaction with the compound of formula (4) to obtain a pegylated 7-dehydrocholesterol derivative of formula (2A).

In addition, the compound of formula (6) may be subject to condensation reaction with the compound of formula (12) to obtain the compound of formula (13). Then, the compound of formula (13) may be subject to deprotection to obtain the compound of formula (14), and the compound of formula (14) may be subject to condensation reaction with the compound of formula (5) to obtain a pegylated 7-dehydrocholesterol derivative of formula (2B).

Further, in some cases, a compound having a protecting group, such as the compound of formula (11), may be optionally used, and in the case that $Y_1$ is an amino acid residue, it may be substituted or unsubstituted with a protecting group.

[Reaction Scheme 3]

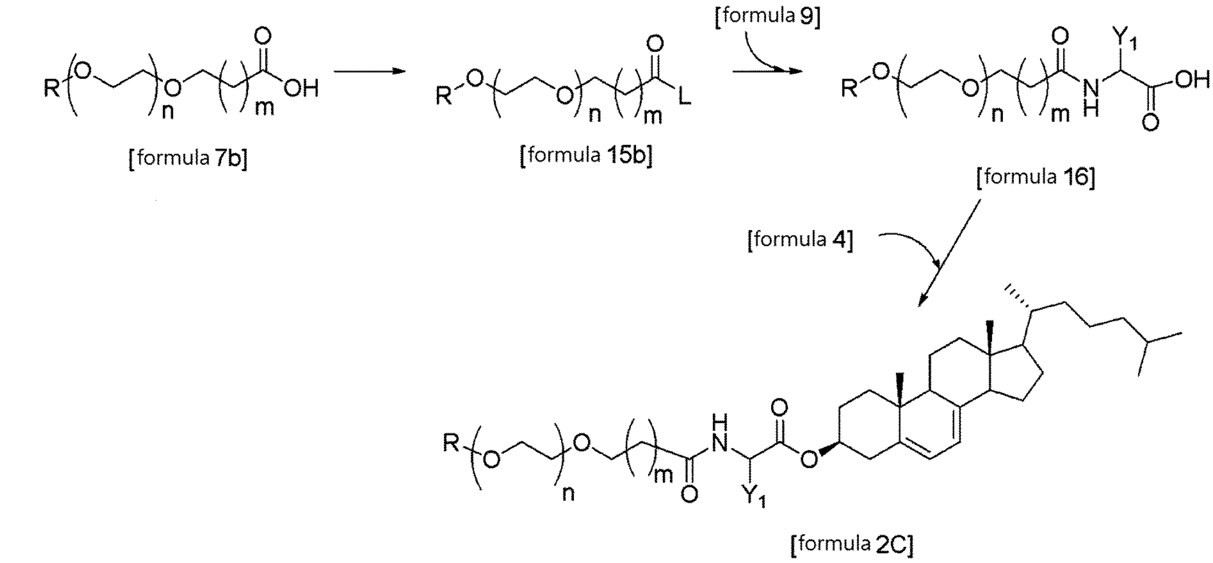

[formula 7b] [formula 15b] [formula 9] [formula 16] [formula 4] [formula 2C]

wherein,

R, n and m are the same as defined in the above formula (I),
L is halogen, succinimide or imidazole, and
$Y_1$ is hydrogen, $C_1$-$C_6$ alkyl or an amino acid residue.

According to Reaction Scheme 3, the compound of formula (7b) may be activated to obtain the compound of formula (15b), which is subject to reaction with the compound of formula (9), to obtain the polyethylene glycol derivative of formula (16). The compound of formula (16) may be subject to condensation reaction with the compound of formula (4) to obtain a pegylated 7-dehydrocholesterol derivative of formula (2C).

[Reaction Scheme 4]

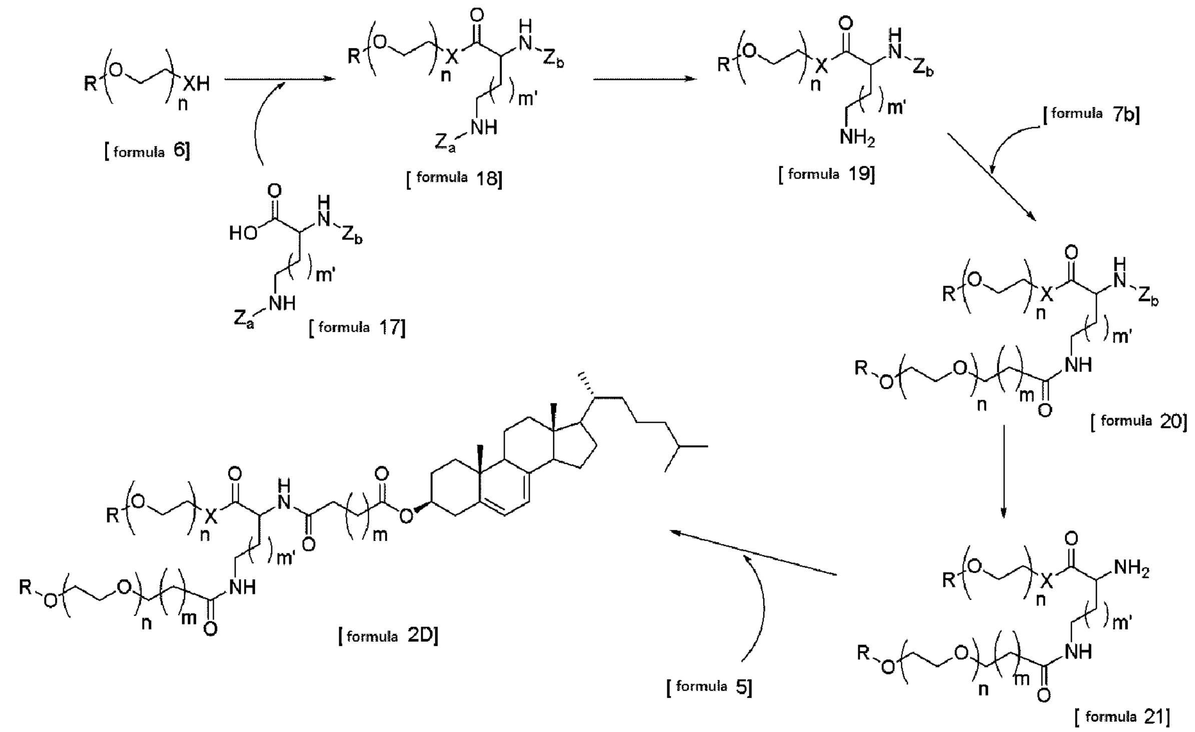

[formula 6] [formula 17] [formula 18] [formula 19] [formula 7b] [formula 20] [formula 21] [formula 5] [formula 2D]

wherein,

R, X, n, m and m' are the same as defined in the above formula (I),
L is halogen, succinimide or imidazole, and
$Z_a$ and $Z_b$ are each a protecting group.

According to Reaction Scheme 4, the compound of formula (6) may be subject to reaction with the compound of formula (17) where it is substituted or unsubstituted with a protecting group, to obtain the compound of formula (18). The compound of formula (18) may be subject to deprotection to obtain the compound of formula (19), which is subject to reaction with the compound of formula (7b), to obtain the compound of formula (20) being a polyethylene glycol derivative of a side chain form. The compound of formula (20) may be subject to deprotection to obtain the compound of formula (21), which is subject to condensation reaction with the compound of formula (5), to obtain a pegylated 7-dehydrocholesterol derivative of formula (2D).

[Reaction Scheme 5]

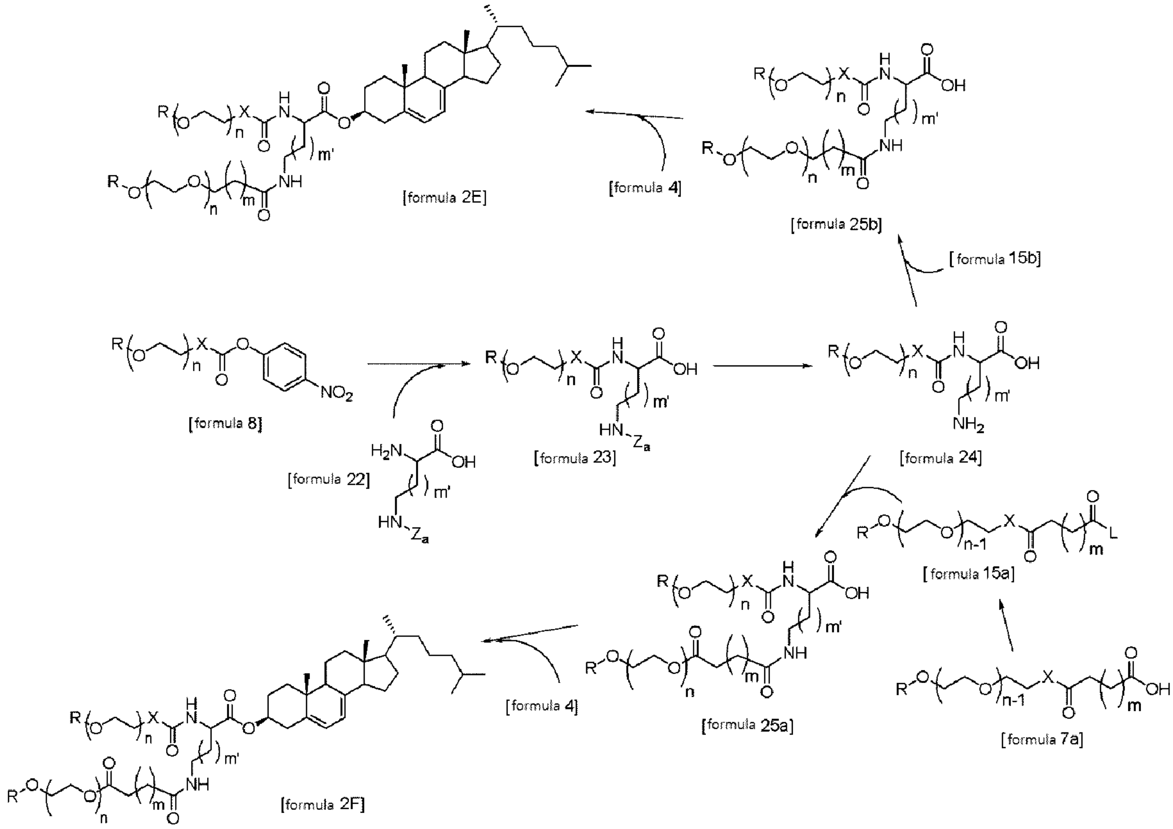


wherein,

R, X, n, m and m' are the same as defined in the above formula (I), and
$Z_a$ is a protecting group.

According to Reaction Scheme 5, the compound of formula (8) may be subject to reaction with the compound of formula (22) where it is substituted or unsubstituted with a protecting group, to obtain the compound of formula (23). The compound of formula (23) may be subject to deprotection to obtain the compound of formula (24), which is subject to reaction with the compound of formula (15b), to obtain the compound of formula (25b). The compound of formula (25b) may be subject to condensation reaction with the compound of formula (4), to obtain a pegylated 7-dehydrocholesterol derivative of formula (2E).

Also, the compound of formula (24) may be subject to reaction with the compound of formula (15a) obtained

by activating the compound of formula (7a) to obtain the compound of formula (25a), and the compound of formula (25a) may be subject to condensation reaction with the compound of formula (4) to obtain a pegylated 7-dehydrocholesterol derivative of formula (2F).

[Reaction Scheme 6]

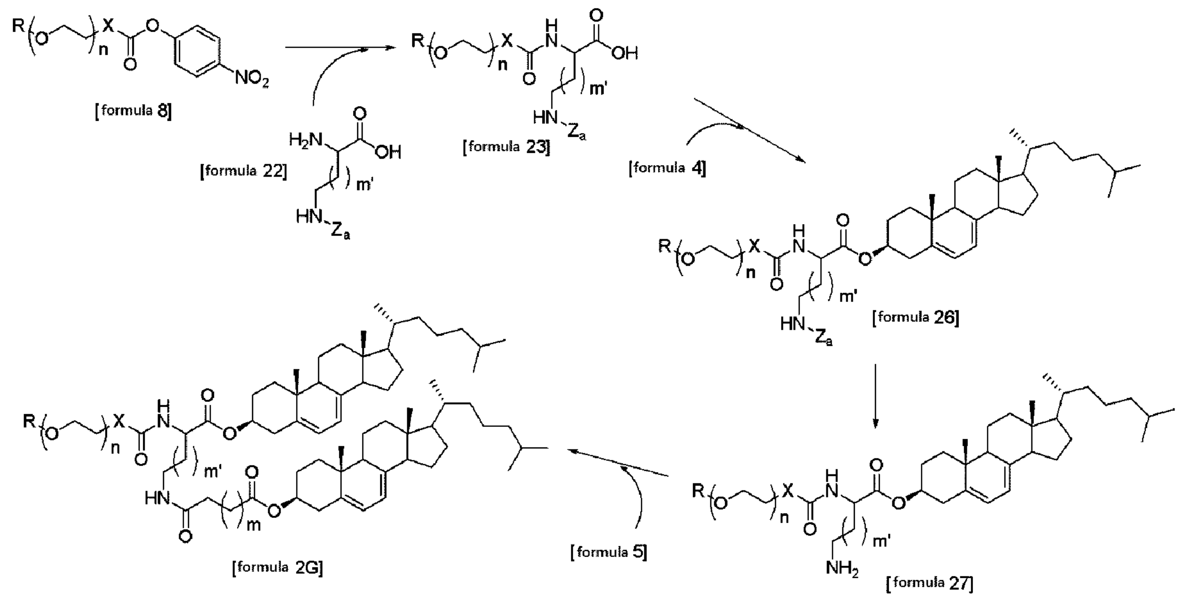


wherein,

R, X, n, m and m' are the same as defined in the above formula (I), and
$Z_a$ is a protecting group.

According to Reaction Scheme 6, the compound of formula (8) may be subject to reaction with the compound of formula (22) where it is substituted or unsubstituted with a protecting group, to obtain the compound of formula (23), and the compound of formula (23) may be subject to condensation reaction with the compound of formula (4) to obtain the compound of formula (26), which is subject to deprotection to obtain the compound of formula (27). The compound of formula (27) may be subject to condensation reaction with the compound of formula (5) to obtain a pegylated 7-dehydrocholesterol derivative of formula (2G).

In the Reaction Schemes, the condensation reaction is preferably carried out in a solvent in the presence of a condensing agent and an organic amine catalyst.

Examples of the condensing agent may include N,N,N',N'-tetramethyl-(benzotriazol-1-yl)-uronium tetrafluoroborate (TBTU), N-(3-dimethylaminopropyl)-N'-ethyl-carbodiimide (EDC), N,N'-diisopropylcarbodiimide (DIC) and N,N'-dicyclohexylcarbodiimide (DCC), but are not limited thereto.

Also, examples of the organic amine catalyst may include diisopropylethylamine (DIPEA) and 4-dimethylaminopyridine (DMAP), but are not limited thereto.

Examples of the solvent may include anhydrous organic solvents, for example at least one selected from dichloromethane, benzene, toluene, tetrahydrofuran and diethyl ether, but are not limited thereto. Also, water or a buffer solution may be used as the solvent, or an organic solvent miscible with water, e.g., acetonitrile, dimethylformamide and the like may be used in combination with water.

The above reactions may be carried out at cooled or warmed temperature.

Meanwhile, examples of the protecting group may include Boc, Fmoc, CBz, tBuCO, Trt, Me, Et and the like, but are not limited thereto.

Also, the deprotection reaction may be carried out in an organic solvent in the presence of an acid or a base. Examples of the organic solvent may include tetrahydrofuran (THF), dichloromethane (DCM), methanol, dimethylformamide (DMF) and the like, but are not limited thereto. Also, examples of the acid or the base may include trifluoroacetic acid (TFA), hydrochloric acid, sulfuric acid, acetic acid, piperidine, sodium hydroxide and the like, but are not limited thereto.

In Reaction Schemes 3 and 5, the substitution reaction of the activated leaving group may be carried out by reaction with thionylchloude, oxalylchloride, N-hydroxysuccinimide, carbonyldiimidazole and the like using an organic amine catalyst, if necessary, in an anhydrous organic solvent.

The pegylated 7-dehydrocholesterol derivative of formula (I) according to the present invention have increased solubility in an aqueous solution and surprisingly improved stability to light and heat, as compared with 7-dehydrocholesterol.

Therefore, the pegylated 7-dehydrocholesterol derivative of formula (I) according to the present invention can be hydrolyzed *in vivo* to act as a source for supplying a precursor of vitamin D, 7-dehydrocholesterol, and thus can be effectively used in a cosmetic composition, a pharmaceutical composition and a functional food for wrinkle alleviation and anti-aging.

The present invention also relates to a cosmetic composition for wrinkle alleviation and anti-aging, comprising the pegylated 7-dehydrocholesterol derivative of formula (I).

The cosmetic composition according to the present invention may comprise the pegylated 7-dehydrocholesterol derivative of formula (I) in an amount of about 0.0001 to 10 wt%, preferably 0.01 to 1 wt% as an active ingredient. The amount of the active ingredient may be determined depending on the purpose of use.

The cosmetic composition of the present invention may include cosmetic ingredients usually used in the art, for example, common adjuvants such as anti-oxidant, stabilizer, solubilizer, vitamin, pigment and perfume, and carriers, in addition to the pegylated 7-dehydrocholesterol derivative of formula (I).

The cosmetic composition of the present invention may be formulated as any form usually used in the art, for example, solution, suspension, emulsion, paste, gel, cream, powder, spray and the like.

In the case where the cosmetic composition is formulated as paste, cream or gel, the examples of the carriers include animal oil, vegetable oil, wax, paraffin, starch, tragacanth, cellulose derivative, polyethylene glycol, silicon, bentonite, silica, talc, zinc oxide and the like.

In the case where the cosmetic composition is formulated as powder or spray, the examples of the carriers include lactose, talc, silica, aluminum hydroxide, calcium silicate, polyamide powder and the like. In particular, in the case where the form is spray, the cosmetic composition can further include propellant such as chlorofluorohydrocarbon, propane/butane and dimethyl ether.

In the case where the cosmetic composition is formulated as solution or emulsion, the examples of the carriers include solvent, solubilizer and emulsifier such as water, ethanol, isopropanol, ethyl carbonate, ethyl acetate, benzyl alcohol, benzyl benzoate, propylene glycol, 1,3-butylglycol oil, glycerol fatty acid ester, polyethylene glycol, sorbitan fatty acid ester and the like.

In the case where the cosmetic composition is formulated as suspension, the examples of the carriers include liquid diluent such as water, ethanol and propylene glycol, suspending agent such as ethoxylated isostearyl alcohol, polyoxyethylene sorbitol ester and polyoxyethylene sorbitan ester, microcrystalline cellulose, aluminum methahydroxide, bentonite, agar, tragacanth and the like.

The cosmetic composition of the present invention may be applied to cosmetics such as skin, lotion, cream, essence, face pack, foundation, coloring cosmetics, sun block cream, two-way cake, face powder, compact, makeup base, makeup cover, eye shadow, lip stick, lip gloss, lip fix and eyebrow pencil.

The present invention further relates to a pharmaceutical composition for wrinkle alleviation and anti-aging, comprising the pegylated 7-dehydrocholesterol derivative of formula (I).

The pharmaceutical composition according to the present invention may be administered via oral (e.g., taking through mouth or inhalation) or parenteral (e.g., injection, transdermal absorption, intrarectal administration) routes, and the injection may be intravenous, subcutaneous, intramuscular or intraperitoneal injection. The pharmaceutical composition according to the present invention may be formulated in the form of tablet, capsule, granule, fine subtilae, powder, sublingual table, suppository, ointment, injection, emulsion, suspension, syrup, spray and the like. These several formulations of the pharmaceutical composition according to the present invention may be prepared using a pharmaceutically acceptable carrier which is conventionally used in the art by the known methods. Examples of the pharmaceutically acceptable carrier may include excipients, binders, disintegrating agents, lubricants, preservatives, antioxidants, isotonic agents, buffering agents, coating agents, sweeteners, solubilizers, bases, dispersing agents, wetting agents, suspending agents, stabilizers, coloring agents and the like.

Although varied depending on the types of the formulations, the pharmaceutical composition according to the present invention comprises the pegylated 7-dehydrocholesterol derivative of formula (I) in an amount of about 0.1 to 10 wt%, preferably 0.1 to 1 wt%.

The specific dosage of the pharmaceutical composition of the present invention may be determined depending on the kinds, weight, sex, severity of disease of mammals to be treated, including a human, the physician's decision and the like. Preferably, a daily dosage for oral administration may range from 5 to 60 $\mu g$ based on a subject weighing 60 kg. The total daily dosage may be administered in a single dose or divided doses, depending on the severity of diseases, the physician's decision and other conditions.

Furthermore, the present invention relates to a functional food for wrinkle alleviation and anti-aging, comprising the pegylated 7-dehydrocholesterol derivative of formula (I).

The kinds of the functional food according to the present invention are not particularly limited. For example, the functional food may be in the form of oral preparations such as powder, granule, tablet, capsule, suspension, emulsion, syrup, or it may be added to general foods such as candies, cookies, chewing gums, ice cream, noodles, breads, beverages and the like.

The functional food of the present invention may be prepared by a conventional method according to its form, and it may suitably contain a carrier being acceptable as a food material, e.g., filling agents, extenders, binders, wetting agents, disintegrating agents, sweeteners, flavoring agents, preservatives, surfactants, lubricants, excipients and the like.

Although varied depending on the kinds of the functional food, the functional food may comprise the pegylated 7-dehydrocholesterol derivative of formula (I) in an amount of about 0.0001 to 10 wt%, preferably 0.1 to 1 wt%.

[Advantageous Effects]

The pegylated 7-dehydrocholesterol derivatives according to the present invention have improved stability and solubility in water and show reduced toxicity.

Therefore, the pegylated 7-dehydrocholesterol derivatives according to the present invention can be effectively used as a good source for supplying vitamin D in a cosmetic composition, a pharmaceutical composition and a functional food for wrinkle alleviation and anti-aging.

Also, the pegylated 7-dehydrocholesterol derivatives according to the present invention can be converted into an active vitamin D by several enzymes *in vivo,* thereby continuously providing bioactivities such as anti-oxidation, wrinkle alleviation and whitening.

[Brief Description of the Drawings]

Fig. 1 is a graph showing thermal stability for the compound (MP350DHC) of Example 1 and 7-dehydrocholesterol (DHC) as a comparative compound at constant temperature of 40 °C.

Fig. 2 is a graph showing thermal stability for the compound (MP350DHC) of Example 1 and 7-dehydrocholesterol (DHC) as a comparative compound at constant temperature of 70 °C.

Fig. 3 is a graph showing photostability for the compound (MP350DHC) of Example 1 and 7-dehydrocholesterol (DHC) as a comparative compound.

[Best Mode]

The present invention is further illustrated by the following examples, which are not to be construed to limit the scope of the invention.

**Example 1: Preparation of mPEG350-S-DHC ester (compound of formula (1A) wherein R is methyl, X is O, n is 7.25 (mean value), and m is 1)**

**Example 1-1: Preparation of 7-dehydrocholesterol succinate (compound of formula (5) wherein m is 1)**

7-Dehydrocholesterol (DHC) of formula (4) (6.45 g, 16.8 mmol) was dissolved in 120 ml of dichloromethane, to which triethylamine (3.39 g, 33.5 mmol) and 4-dimethylaminopyridine (0.82 g, 6.7 mmol) were added to give a solution. Thereto, succinic anhydride (compound of formula (3) wherein m is 1) (3.35 g, 33.5 mmol) was added, followed by stirring at room temperature for 12 hours. The reaction solution was successively washed with 3.5% HCl aqueous solution and saturated aqueous sodium bicarbonate solution and saturated aqueous sodium chloride solution. Then, the organic phase was dried over anhydrous magnesium sulfate, filtered, concentrated under reduced pressure. The residue was purified by silica gel column chromatography (ethyl acetate:hexane = 1:2) to give 7.2 g of the target compound.
$^1$H NMR 400 MHz($CDCl_3$) $\delta$ 5.59(dd, 1H), 5.41(dd, 1H), 4.75(m, 1H), 2.72(dt, 2H), 2.64(dt, 2H), 2.52(d, 1H), 2.39(t, 1H), 2.18-1.85(m, 6H), 1.82-1.50(m, 6H), 1.48-0.93(m, 11H), 0.97(s, 6H), 0.90(d, 3H), 0.89(d, 3H) 0.64(s, 3H)

**Example 1-2: Preparation of mPEG350-S-DHC ester (compound of formula (1A) wherein R is methyl, X is O, n is 7.25 (mean value), and m is 1)**

The 7-dehydrocholesterol succinate given in Example 1-1 (compound of formula (5) wherein m is 1) (1.1 g, 2.30 mmol) and mPEG350-OH (compound of formula (6) wherein R is methyl, n is 7.25 (mean value), and X is O) (0.8 g, 2.30 mmol) were dissolved in 10 ml of dichloromethane, to which 4-dimethylaminopyridine (0.42 g, 3.44 mmol) was added. Thereto, N-(3-dimethylaminopropyl)-N'-ethyl-carbodiimide (0.88 g, 4.59 mmol) was slowly added, followed by stirring at room temperature for 12 hours. The reaction solution was successively washed with 1.0 N HCl aqueous solution, saturated aqueous sodium bicarbonate solution, and saturated aqueous sodium chloride solution. Then, the organic phase was dried over anhydrous magnesium sulfate, filtered, concentrated under reduced pressure, and dried under vacuum to give 1.77 g of the target compound.
$^1H$ NMR 400 MHz($CDCl_3$) $\delta$ 5.59(dd, 1H), 5.40(dd, 1H), 4.74(m, 1H), 4.27(t, 2H), 3.72(t, 2H), 3.67(PEG backbone), 3.57(t, 2H), 3.40(s, 3H), 2.67(dt, 2H), 2.62(dt, 2H), 2.52(d, 1H), 2.38(t, 1H), 2.12-1.89(m, 6H), 1.75-1.53(m, 6H), 1.43-0.95(m, 11H), 0.97(s, 6H), 0.90(d, 3H), 0.88(d, 3H) 0.64(s, 3H)

**Example 2: Preparation of mPEG2K-S-DHC ester (compound of formula (1A) wherein R is methyl, X is O, n is 44.3 (mean value), and m is 1)**

The procedure of Example 1-2 was repeated except for using 7-dehydrocholesterol succinate given in Example 1-1 (82.8 mg, 0.22 mmol), mPEG2K-OH (compound of formula (6) wherein R is methyl, n is 44.3 (mean value), and X is O) (452 mg, 0.22 mmol), 5 ml of dichloromethane, 4-dimethylaminopyridine (65.7 mg, 0.54 mmol), and N-(3-dimethylaminopropyl)-N'-ethyl-carbodiimide (123.8 mg, 0.65 mmol), to give 462 mg of the target compound.
$^1H$ NMR 400 MHz($CDCl_3$) $\delta$ 5.57(m, 1H), 5.40(m, 1H), 4.72(m, 1H), 4.25(t, 2H), 3.83-3.45(PEG backbone), 3.38(s, 3H), 2.70-2.56(m, 5H), 2.48(dt, 1H), 2.38(dt, 1H), 2.12-1.89(m, 6H), 1.75-1.53(m, 6H), 1.43-0.95(m, 11H), 0.95(s, 6H), 0.88(d, 3H), 0.84(d, 3H) 0.62(s, 3H)

**Example 3: Preparation of mTrEG-S-DHC ester (compound of formula (1A) wherein R is methyl, X is O, n is 3, and m is 1)**

7-Dehydrocholesterol of formula (4) (0.54 g, 1.40 mmol) and methoxy triethylene glycol succinate (compound of formula (7a) wherein R is methyl, n is 3, X is O, and m is 1; mTrEG-succinic acid) (0.37 g, 1.40 mmol) were dissolved in 8 ml of dichloromethane, to which 4-dimethylaminopyridine (0.26 g, 2.11 mmol) was added. Thereto, N-(3-dimethylaminopropyl)-N'-ethyl-carbodiimide (0.67 g, 3.51 mmol) was slowly added, followed by stirring at room temperature for 12 hours. The reaction solution was successively washed with 1.0 N HCl aqueous solution, saturated aqueous sodium bicarbonate solution, and saturated aqueous sodium chloride solution. Then, the organic phase was dried over anhydrous magnesium sulfate, filtered, concentrated under reduced pressure, and dried under vacuum, to give 0.69 g of the target compound.
$^1H$ NMR 400 MHz($CDCl_3$) $\delta$ 5.58(dd, 1H), 5.40(dd, 1H), 4.74(m, 1H), 4.28(t, 2H), 3.72(t, 2H), 3.68(m, 6H), 3.59(t, 2H), 3.40(s, 3H), 2.65(m, 4H), 2.52(dd, 1H), 2.39(t, 1H), 2.12-1.89(m, 6H), 1.75-1.53(m, 6H), 1.43-0.95(m, 11H), 0.97(s, 6H), 0.90(d, 3H), 0.88(d, 3H) 0.64(s, 3H)

**Example 4: Preparation of mPEG350-S-DHC amide (compound of formula (1A) wherein R is methyl, X is NH, n is 7.25 (mean value), and m is 1)**

The 7-dehydrocholesterol succinate given in Example 1-1 (346.2 mg, 0.714 mmol) and mPEG350-$NH_2$ (compound of formula (6) wherein R is methyl, n is 7.25 (mean value), and X is NH; average molecular weight 350) (250 mg, 0.714 mmol) were dissolved in 6 ml of dichloromethane, to which 4-dimethylaminopyridine (87.3 mg, 0.714 mmol) was added. Thereto, N-(3-dimethylaminopropyl)-N'-ethyl-carbodiimide (410.8 g, 2.14 mmol) was slowly added, followed by stirring at room temperature for 12 hours. The reaction solution was successively washed with 1.0 N HCl aqueous solution, saturated aqueous sodium bicarbonate solution, and saturated aqueous sodium chloride solution. Then, the organic phase was dried over anhydrous magnesium sulfate, filtered, concentrated under reduced pressure, and dried under vacuum, to give 508 mg of the target compound.
$^1H$ NMR 400 MHz($CDCl_3$) $\delta$ 6.35(s, 1H), 5.57(dd, 1H), 5.39(dd, 1H), 4.72(m, 1H), 3.66(PEG back-bone), 3.47(t, 2H), 3.39(s, 3H), 2.65(dt, 2H), 2.48(d, 1H), 2.37(t, 1H), 2.08-1.89(m, 6H), 1.74-1.50(m, 6H), 1.42-0.96(m, 11H), 0.96(s, 6H), 0.88(d, 3H), 0.87(s, 3H), 0.63(s, 3H)

**Example 5: Preparation of mPEG2K-P-DHC (compound of formula (1B) wherein R is methyl, n is 44.3 (mean value), and m is 1)**

7-Dehydrocholesterol of formula (4) (0.32 g, 0.84 mmol) and mPEG2K-propionic acid (compound of formula (7b) wherein R is methyl, n is 44.3 (mean value), and m is 1; average molecular weight 2,000) (1.68 g, 0.84 mmol) were dissolved in 100 ml of dichloromethane, to which 4-dimethylaminopyridine (0.154 g, 1.26 mmol) was added. Thereto, N-(3-dimethylaminopropyl)-N'-ethyl-carbodiimide (0.322 g, 1.68 mmol) was slowly added, followed by stirring at room temperature for 12 hours. The reaction solution was successively washed with 1.0 N HCl aqueous solution, saturated aqueous sodium bicarbonate solution, and saturated aqueous sodium chloride solution. Then, the organic phase was dried over anhydrous magnesium sulfate, filtered, concentrated under reduced pressure, and dried under vacuum, to give 1.57g of the target compound.
$^1$H NMR 400 MHz($CDCl_3$) $\delta$ 5.58(m, 1H), 5.40(m, 1H), 4.74(m, 1H), 3.84-3.47(PEG backbone), 3.39(s, 3H), 2.67(dt, 2H), 2.60(dt, 2H), 2.52(d, 1H), 2.38(t, 1H), 2.12-1.89(m, 6H), 1.75-1.53(m, 6H), 1.43-0.95(m, 11H), 0.96(s, 6H), 0.89(d, 3H), 0.87(d, 3H) 0.63(s, 3H)

**Example 6: Preparation of mPEG2K-O-A-DHC (compound of formula (2A) wherein R is methyl, X is O, n is 44.3 (mean value), and $Y_1$ is methyl)**

**Example 6-1: Preparation of mPEG2K-NPC (the compound of formula (8) wherein R is methyl, X is O, n is 44.3(mean value))**

Dried mPEG2K-OH (compound of formula (6) wherein R is methyl, n is 44.3 (mean value), and X is O) (30 g, 14.9 mmol) was dissolved in 120 ml of dichloromethane to give a solution, to which 4-nitrophenyl chloroformate (12 g, 59.7 mmol) and pyridine (5.9 ml, 74.6 mmol) were added, followed by stirring at room temperature for 20 hours. The reaction solution was successively washed with 150 ml of 1N HCl aqueous solution and 150 ml of saturated aqueous sodium chloride solution. Then, the organic phase was dried over anhydrous magnesium sulfate, filtered, and concentrated under reduced pressure. The resulting mixture was precipitated into diethyl ether to give 33.5 g of the target compound as a white solid.

**Example 6-2: Preparation of mPEG2K-Ala-OH (compound of formula (10) wherein R is methyl, X is O, n is 44.3 (mean value), and $Y_1$ is methyl)**

mPEG2K-NPC given in Example 6-1 (16 g, 7.2 mmol) was dissolved in 100 ml of acetonitrile and 60 ml of distilled water to give a solution. Thereto, H-Ala-OH (compound of formula (9) wherein $Y_1$ is methyl) (1.9 g, 21.7 mmol), diisopropylethylamine (1.5 ml, 21.7 mmol) were added, followed by stirring at room temperature for 8 hours. The reaction solution was extracted with 300 ml of dichloromethane and successively washed with 300 ml of 1N HCl aqueous solution and 300 ml of saturated aqueous sodium chloride solution. Then, the organic phase was dried over anhydrous magnesium sulfate, filtered, and concentrated under reduced pressure. The resulting mixture was precipitated into diethyl ether, to give the target compound (18.8 g, 36.4 mmol) as a white solid.

**Example 6-3: Preparation of mPEG2K-O-A-DHC (compound of formula (2A) wherein R is methyl, X is O, n is 44.3 (mean value), and $Y_1$ is methyl)**

7-Dehydrocholesterol of formula (4) (0.2 g, 0.48 mmol) and mPEG2K-Ala-OH given in Example 6-2 (0.10 g, 0.23 mmol) were dissolved in 100 ml of dichloromethane, to which 4-dimethylaminopyridine (0.14 g, 1.14 mmol) was added. Thereto, N-(3-dimethylaminopropyl)-N'-ethyl-carbodiimide (0.2 g, 1.14 mmol) was slowly added, followed by stirring at room temperature for 12 hours. The reaction solution was successively washed with 1.0 N HCl aqueous solution, saturated aqueous sodium bicarbonate solution, and saturated aqueous sodium chloride solution. Then, the organic phase was dried over anhydrous magnesium sulfate, filtered, concentrated under reduced pressure, and dried under vacuum, to give 1.06g of the target compound.
$^1$H NMR 400 MHz($CDCl_3$) $\delta$ 5.59(dd, 1H), 5.44(dd, 1H), 5.41(bs, 1H), 4.36(q, 1H), 4.22(t, 2H), 3.83(t, 2H), 3.77-3.52(PEG back-bone), 3.48(t, 2H), 3.39(s, 3H), 2.80(dt, 2H), 2.55(d, 2H), 2.41(d, 2H), 2.19-1.77(m, 6H), 1.74-1.52(m, 6H), 1.43-1.08(m, 11H), 0.96(s, 6H), 0.89(d, 3H), 0.88(d, 3H), 0.63(s, 3H)

**Example 7: Preparation of mPEG2K-NH-A-DHC (compound of formula (2B) wherein R is methyl, n is 44.3 (mean value), X is NH, $Y_1$ is methyl, and m is 1)**

**Example 7-1: Preparation of mPEG2K-NH-Ala-Fmoc (compound of formula (13) wherein R is methyl, n is 44.3 (mean value), X is NH, $Y_1$ is methyl, and $Z_b$ is Fmoc)**

mPEG2K-$NH_2$(compound of formula (6) wherein R is methyl, n is 44.3, and X is NH) (280 mg, 0.14 mmol), HOBt (94.6 mg, 0.70 mmol), diisopropylethylamine (90.5 mg, 0.70 mmol) were dissolved in dimethylformamide (DMF) and stirred for 10 minutes to give a solution. Thereto, a solution obtained by mixing Fmoc-Ala-OH (compound of formula (12) wherein $Z_b$ is Fmoc, $Y_1$ is methyl) (218.0 mg, 0.70 mmol) and PyBOP (364.5 mg, 0.70 mmol) and stirring the mixture for 10 minutes was added, followed by stirring at room temperature for 5 hours. The reaction solution was successively washed with 150ml of 1N HCl aqueous solution and 150ml of saturated aqueous sodium chloride solution. Then, the organic phase was dried over anhydrous magnesium sulfate, filtered, and concentrated under reduced pressure. The resulting mixture was precipitated into diethyl ether, to give 301.1 mg of the target compound as a white solid.

**Example 7-2: Preparation of mPEG2K-NH-Ala-H (compound of formula (14) wherein R is methyl, n is 44.3 (mean value), X is NH, and $Y_1$ is methyl)**

mPEG2K-NH-Ala-Fmoc given in Example 7-1 (300 mg, 0.13 mmol) was dissolved in 6 ml of DMF solution containing 25% piperidine therein, followed by stirring at room temperature for 3 hours. The reaction solution was precipitated into 100 ml of diethyl ether, to give 0.26 mg of the target compound as a white solid.

**Example 7-3: Preparation of mPEG2K-NH-A-DHC (compound of formula (2B) wherein R is methyl, n is 44.3 (mean value), X is NH, $Y_1$ is methyl, and m is 1)**

7-Dehydrocholesterol succinate given in Example 1-1 (120 mg, 0.24 mmol) and mPEG2K-NH-Ala-H (250 mg, 0.12 mmol) were dissolved in 5 ml of dichloromethane, to which 4-dimethylaminopyridine (17.7 mg, 0.14 mmol) was added. Thereto, N-(3-dimethylaminopropyl)-N'-ethyl-carbodiimide (69.5 mg, 0.36 mmol) was slowly added, followed by stirring at room temperature for 12 hours. The reaction solution was successively washed with 1.0 N HCl aqueous solution, saturated aqueous sodium bicarbonate solution, and saturated aqueous sodium chloride solution. Then, the organic phase was dried over anhydrous magnesium sulfate, filtered, concentrated under reduced pressure, and dried under vacuum, to give 283 mg of the target compound.
$^1$H NMR 400 MHz($CDC1_3$) $\delta$ 5.59(dd, 1H), 5.44(m, 3H), 4.33(q, 1H), 4.23(m, 1H), 3.66(PEG backbone), 3.48(t, 2H), 3.39(s, 3H), 2.72(dt, 2H), 2.64(dt, 2H), 2.52(d, 1H), 2.48(d, 3H), 2.39(t, 1H), 2.18-1.85(m, 6H), 1.82-1.50(m, 6H), 1.48-0.93(m, 11H), 0.97(s, 6H), 0.90(d, 3H), 0.89(d, 3H) 0.64(s, 3H)

**Example 8: Preparation of mPEG2K-P-Ala-DHC (compound of formula (2C) wherein R is methyl, n is 44.3 (mean value), m is 1, and $Y_1$ is methyl)**

**Example 8-1: Preparation of mPEG2K-P-NHS (compound of formula (15b) wherein R is methyl, n is 44.3 (mean value), m is 1, and L is succinimide)**

mPEG-PA (compound of formula (7b) wherein R is methyl, n is 44.3 (mean value), and m is 1) (1 g, 0.476 mmol) was dissolved in 7 ml of dichloromethane to give a solution. Thereto, N-hydroxysuccinimide (0.27 g, 2.38 mmol), 1-ethyl-3-(3-dimethylaminopropyl)carbodiimide (0.45 g, 2.38 mmol), dimethylaminopyridine (0.18 g, 1.198 mmol) were added, followed by stirring at room temperature for 5 hours. The reaction solution was extracted with 100 ml of dichloromethane, and successively washed with 100 ml of 1N HCl aqueous solution, 100 ml of saturated aqueous sodium bicarbonate solution, and 100 ml of saturated aqueous sodium chloride solution. Then, the organic phase was dried over anhydrous magnesium sulfate, filtered, and concentrated under reduced pressure. The resulting mixture was precipitated into diethyl ether, to give 0.94 g of the target compound as a white solid.

**Example 8-2: Preparation of mPEG2K-P-Ala-OH (compound of formula (16) wherein R is methyl, n is 44.3 (mean value), m is 1, and $Y_1$ is methyl)**

mPEG2K-P-NHS given in Example 8-1 (450 mg, 0.21 mmol) was dissolved in 5 ml of dichloromethane to give a solution. The solution was slowly added to a solution of H-Ala-OH (92.4 mg, 1.04 mmol) and triethylamine (115.5 mg, 1.14 mmol) dissolved in 2.5 ml of dichloromethane and 2.5 ml of dimethylformamide, followed by stirring at room temperature for 3 hours. The reaction solution was successively washed with 1.0 N HCl aqueous solution, saturated aqueous

sodium bicarbonate solution, and saturated aqueous sodium chloride solution. Then, the organic phase was dried over anhydrous magnesium sulfate, filtered, concentrated under reduced pressure, and dried under vacuum, to give 478 mg of the target compound.

**Example 8-3: Preparation of mPEG2K-P-Ala-DHC (compound of formula (2C) wherein R is methyl, n is 44.3 (mean value), m is 1, and $Y_1$ is methyl)**

7-Dehydrocholesterol of formula (4) (90.4 mg, 0.24 mmol) and mPEG2K-P-Ala-OH given in Example 8-2 (420 mg, 0.19 mmol) were dissolved in 10 ml of dichloromethane, to which 4-dimethylaminopyridine (35.9 mg, 0.29 mmol) was added. Thereto, N-(3-dimethylaminopropyl)-N'-ethyl-carbodiimide 93.9 mg(0.49 mmol) was slowly added, followed by stirring at room temperature for 12 hours. The reaction solution was successively washed with 1.0 N HCl aqueous solution, saturated aqueous sodium bicarbonate solution, and saturated aqueous sodium chloride solution. Then, the organic phase was dried over anhydrous magnesium sulfate, filtered, concentrated under reduced pressure, and dried under vacuum, to give 457 mg of the target compound.
$^1H$ NMR 400 MHz($CDCl_3$) $\delta$ 5.59(dd, 1H), 5.44(m, 3H), 4.26(q, 1H), 4.23(m, 1H), 3.66(PEG backbone), 3.39(s, 3H), 2.60(t, 2H), 2.52(d, 1H), 2.39(t, 1H), 2.18-1.85(m, 6H), 1.82-1.50(m, 6H), 1.50(d, 3H), 1.48-0.93(m, 11H), 0.97(s, 6H), 0.90(d, 3H), 0.89(d, 3H) 0.64(s, 3H)

**Example 9: Preparation of α-mPEG2K-NH-γ-mPEG2K-P-Dab-DHC (compound of formula (2D) wherein R is methyl, n is 44.3 (mean value), X is NH, and m and m' are each 1)**

**Example 9-1: Preparation of α-mPEG2K-NH-Dab(tBoc)-Fmoc (compound of formula (18) wherein R is methyl, n is 44.3 (mean value), X is NH, m' is 1, $Z_a$ is a protecting group of tBoc, and $Z_b$ is a protecting group of Fmoc)**

The procedure of Example 7-1 was repeated except for using mPEG2K-$NH_2$ (400 mg, 0.20 mmol), HOBt (135.2 mg, 1.00 mmol), diisopropylethylamine (129.3 mg, 1.00 mmol), Fmoc-Dab(tBoc)-OH (compound of formula (17) wherein m' is 1, $Z_a$ is a protecting group of tBoc, and $Z_b$ is a protecting group of Fmoc) (264.4 mg, 0.60 mmol), PyBOP (520.6 mg, 1.00 mmol), and 40 ml of DMF, to give 473 mg of the target compound as a white solid.

**Example 9-2: Preparation of α-mPEG2K-NH-Dab-Fmoc (compound of formula (19) wherein R is methyl, n is 44.3 (mean value), X is NH, m' is 1, and $Z_b$ is a protecting group of Fmoc)**

α-mPEG2K-NH-Dab(tBoc)-Fmoc given in Example 9-1 (450 mg, 0.47 mmol) was dissolved in 5 ml of dichloromethane to give a solution. Thereto, 5 ml of trifluoroacetic acid was added, followed by stirring at room temperature for 3 hours. The reaction solution was added with 50 ml of dichloromethane, and washed with 50 ml of saturated aqueous sodium bicarbonate solution and 50 ml of saturated aqueous sodium chloride solution. Then, the organic phase was dried over anhydrous magnesium sulfate, filtered, and concentrated under reduced pressure. The resulting mixture was precipitated into diethyl ether, to give 428 mg of the target compound as a white solid.

**Example 9-3: Preparation of α-mPEG2K-NH-γ-mPEG2K-P-Dab-Fmoc (compound of formula (20) wherein R is methyl, n is 44.3 (mean value), X is NH, m and m' are each 1, and $Z_b$ is a protecting group of Fmoc)**

α-mPEG2K-NH-Dab-Fmoc given in Example 9-2 (420 mg, 0.18 mmol) was dissolved in 8 ml of dichloromethane, to which mPEG2K-PA (374.9 mg, 0.18 mmol), 4-dimethylaminopyridine (33.2 mg, 0.27 mmol) were added. Thereto, N-(3-dimethylaminopropyl)-N'-ethyl-carbodiimide (69.4 mg, 0.36 mmol) was slowly added, followed by stirring at room temperature for 12 hours. The reaction solution was successively washed with 1.0 N HCl aqueous solution, saturated aqueous sodium bicarbonate solution, and saturated aqueous sodium chloride solution. Then, the organic phase was dried over anhydrous magnesium sulfate, filtered, concentrated under reduced pressure, and dried under vacuum, to give 750 mg of the target compound.

**Example 9-4: Preparation of α-mPEG2K-NH-γ-mPEG2K-P-Dab-H (compound of formula (21) wherein R is methyl, n is 44.3 (mean value), X is NH, and m and m' are each 1)**

α-mPEG2K-NH-γ-mPEG2K-P-Dab-Fmoc given in Example 9-3 (700 mg, 0.16 mmol) was dissolved in 14 ml of DMF solution containing 25% piperidine therein, followed by stirring at room temperature for 3 hours. The reaction solution was precipitated into 100 ml of diethyl ether, to give 651 mg of the target compound as a white solid.

**Example 9-5: Preparation of α-mPEG2K-NH-γ-mPEG2K-P-Dab-DHC (compound of formula (2D) wherein R is methyl, n is 44.3 (mean value), X is NH, and m and m' are each 1)**

The procedure of Example 7-3 was repeated except that 7-dehydrocholesterol succinate given in Example 1-1 (64.2 mg, 0.13 mmol) and α-mPEG2K-NH-γ-mPEG2K-P-Dab-H given in Example 9-4 (550 mg, 0.13 mmol) were dissolved in 10 ml of dichloromethane, to which 4-dimethylaminopyridine (24.3 mg, 0.20 mmol) was added, and N-(3-dimethylaminopropyl)-N'-ethyl-carbodiimide (50.8 mg, 0.26 mmol) was used, to give 587 mg of the target compound.
$^1$H NMR 400 MHz($CDCl_3$) δ 5.74(m, 2H), 5.59(d, 1H), 5.44(m, 2H), 4.35(m, 1H), 4.23(m, 3H), 3.84-3.66(PEG back-bone), 3.57(m, 6H), 3.48(t, 2H), 3.39(s, 6H), 2.72(dt, 2H), 2.64(dt, 2H), 2.62(t, 2H), 2.53-2.39(m, 4H), 2.20-1.52(m, 16H), 1.41-1.06(m, 11H), 0.96(s, 6H), 0.89(d, 3H), 0.88(d, 3H), 0.63(s, 3H)

**Example 10: Preparation of α-mPEG2K-γ-mPEG2K-P-Dab-DHC (compound of formula (2E) wherein R is methyl, n is 44.3(mean value), X is O, and m and m' are each 1)**

**Example 10-1: Preparation of mPEG2K-Dab(tBoc)-OH (compound of formula (23) wherein R is methyl, n is 44.3(mean value), X is O, m' is 1, and $Z_a$ is tBoc)**

The procedure of Example 6-2 was repeated except for using a solution of mPEG2K-NPC given in Example 6-1 (2 g, 0.91 mmol) dissolved in 15 ml of acetonitrile and 10 ml of distilled water, H-Dab(tBoc)-OH (compound of formula (22) wherein m' is 1, and $Z_a$ is tBoc) (0.59 g, 2.72 mmol), and diisopropylethylamine (0.46 ml, 2.72 mmol), to give 1.67 g of the target compound.

**Example 10-2: Preparation of mPEG2K-Dab-OH (compound of formula (24) wherein R is methyl, n is 44.3(mean value), X is O, and m' is 1)**

The procedure of Example 9-2 was repeated except that mPEG2K-Dab(tBoc)-OH given in Example 10-1 (1 g, 0.47 mmol) was dissolved in 10 ml of dichloromethane, to which trifluoroacetic acid (10 ml, 0.13 mmol) was added, to give 0.76 g of the target compound.

**Example 10-3: Preparation of α-mPEG2K-γ-mPEG2K-P-Dab-OH (compound of formula (25b) wherein R is methyl, n is 44.3 (mean value), X is O, and m and m' are each 1)**

mPEG2K-Dab-OH given in Example 10-2 (0.34 g, 0.16 mmol) and mPEG2K-P-NHS given in Example 8-1 (0.35 g, 0.16 mmol) were dissolved in 5 ml of dichloromethane to give a solution. Thereto, 87 μl of triethylamine was added, followed by stirring at room temperature for 12 hours. The reaction solution was added with 100 ml of dichloromethane and successively washed with 100 ml of 1N HCl aqueous solution and 100 ml of saturated aqueous sodium chloride solution. Then, the organic phase was dried over anhydrous magnesium sulfate, filtered, and concentrated under reduced pressure. The resulting mixture was precipitated into diethyl ether, to give 0.54 g of the target compound as a white solid.

**Example 10-4: Preparation of α-mPEG2K-γ-mPEG2K-P-Dab-DHC (compound of formula (2E) wherein R is methyl, n is 44.3 (mean value), X is O, and m and m' are each 1)**

7-Dehydrocholesterol of formula (4) (38.0 mg, 0.098 mmol) and α-mPEG2K-γ-mPEG2K-P-Dab-OH given in Example 10-3 (0.10 g, 0.049 mmol) were dissolved in 50 ml of dichloromethane, to which 4-dimethylaminopyridine (6.0 mg, 0.247 mmol) was added. Thereto, N-(3-dimethylaminopropyl)-N'-ethyl-carbodiimide (95 mg, 0.494 mmol) was slowly added, followed by stirring at room temperature for 12 hours. The reaction solution was successively washed with 1.0 N HCl aqueous solution, saturated aqueous sodium bicarbonate solution, and saturated aqueous sodium chloride solution. Then, the organic phase was dried over anhydrous magnesium sulfate, filtered, concentrated under reduced pressure, and dried under vacuum, to give 0.48 g of the target compound.
$^1$H NMR 400 MHz($CDCl_3$) δ 6.85(bs, 1H), 5.74(m, 1H), 5.59(d, 1H), 5.32(d, 1H), 4.35(m, 1H), 4.23(m, 3H), 3.84-3.66(PEG back-bone), 3.49(t, 2H), 3.39(s, 6H), 2.53-2.39(m, 4H), 2.20-1.52(m, 8H), 1.41-1.06(m, 11H), 0.96(s, 6H), 0.89(d, 3H), 0.88(d, 3H), 0.63(s, 3H)

**Example 11: Preparation of α-mPEG5K-γ-mPEG5K-S-Dab-DHC (compound of formula (2F) wherein R is methyl, n is 113.0 (mean value), X is O, and m and m' are each 1)**

**Example 11-1: Preparation of mPEG5K-S-NHS (compound of formula (15a) wherein R is methyl, n is 113.0 (mean value), X is O, m is 1, and L is NHS)**

The procedure of Example 8-1 was repeated except for using mPEG5K-SA (compound of formula (7a) wherein R is methyl, n is 113.0 (mean value), X is O, and m is 1) (1.0 g, 0.20 mmol), N-hydroxysuccinimide (0.16 g, 1.4 mmol), dimethylaminopyridine (73 mg, 0.6 mmol), 1-ethyl-3-(3-dimethylaminopropyl)carbodiimide (0.27 g, 1.4 mmol), and 7 ml of dichloromethane, to give 0.93 g of target compound as a white solid.

**Example 11-2: Preparation of mPEG5K-Dab(tBoc)-OH (compound of formula (23) wherein R is methyl, n is 113.0 (mean value), X is O, m' is 1, and $Z_a$ is tBoc)**

The procedure of Example 10-1 was repeated except that mPEG5K-NPC (2 g, 0.384 mmol) was dissolved in 10 ml of acetonitrile and 10 ml of distilled water, to which H-Dab(tBoc)-OH (0.17 g, 0.768 mmol) and diisopropylethylamine (0.2 ml, 1.15 mmol) were added, to give 1.7 g of the target compound.

**Example 11-3: Preparation of mPEG5K-Dab-OH (compound of formula (24) wherein R is methyl, n is 113.0 (mean value), X is O, and m' is 1)**

The procedure of Example 9-2 was repeated except for using mPEG5K-Dab(tBoc)-OH given in Example 11-2 (1.3 g, 0.254 mmol), 13 ml of trifluoroacetic acid and 13 ml of dichloromethane, to give 1.2 g of the target compound.

**Example 11-4: Preparation of α-mPEG5K-γ-mPEG5K-S-Dab-OH (compound of formula (25a) wherein R is methyl, n is 113.0 (mean value), X is O, and m and m' are each 1)**

mPEG5K-Dab-OH given in Example 11-3 (0.79 g, 0.15 mmol) and mPEG5K-S-NHS given in Example 11-1 (0.8 g, 0.15 mmol) were dissolved in 15 ml of dichloromethane to give a solution. Thereto, 0.1 ml of triethylamine was added, followed by stirring at room temperature for 12 hours. The reaction solution was added with 50 ml of dichloromethane and successively washed with 50 ml of 1N HCl aqueous solution and 50 ml of saturated aqueous sodium chloride solution. Then, the organic phase was dried over anhydrous magnesium sulfate, filtered, and concentrated under reduced pressure. The resulting mixture was precipitated into diethyl ether, to give 1.35 g of the target compound as a white solid.

**Example 11-5: Preparation of α-mPEG5K-γ-mPEG5K-S-Dab-DHC (compound of formula (2F) wherein R is methyl, n is 113.0 (mean value), X is O, and m and m' are each 1)**

7-Dehydrocholesterol of formula (4) (38.0 mg, 0.098 mmol) and α-mPEG5K-γ-mPEG5K-S-Dab-OH given in Example 11-4 (0.5 g, 0.049 mmol) were dissolved in 10 ml of dichloromethane, to which 4-dimethylaminopyridine (6.0 mg, 0.247 mmol) was added. Thereto, N-(3-dimethylaminopropyl)-N'-ethyl-carbodiimide (95 mg, 0.494 mmol) was slowly added, followed by stirring at room temperature for 12 hours. The reaction solution was successively washed with 1.0 N HCl aqueous solution, saturated aqueous sodium bicarbonate solution, and saturated aqueous sodium chloride solution. Then, the organic phase was dried over anhydrous magnesium sulfate, filtered, concentrated under reduced pressure, and dried under vacuum, to give the target compound 0.49 g.
$^1$H NMR 400 MHz($CDCl_3$) δ 6.79(bs, 1H), 5.69(m, 1H), 5.53(d, 1H), 5.30(d, 1H), 4.29(m, 1H), 4.17(m, 3H), 3.91-3.50(PEG backbone), 3.45(t, 4H), 3.34(t, 4H), 3.34(s, 6H), 2.55-2.48(m, 8H), 2.1-1.62(m, 14H), 1.42-1.14(m, 11H), 0.95(s, 6H), 0.84(d, 3H), 0.82(d, 3H), 0.59(s, 3H)

**Example 12: Preparation of α-mPEG350-γ-DHC-Dab-DHC (compound of formula (2G) wherein R is methyl, n is 7.25 (mean value), X is O, and m and m' are each 1)**

**Example 12-1: Preparation of mPEG350-NPC (compound of formula (8) wherein R is methyl, X is O, and n is 7.25 (mean value))**

Dried mPEG350-OH (compound of formula (6) wherein R is methyl, n is 7.25 (mean value), and X is O) (15 g, 42.86 mmol) was dissolved in 200 ml of dichloromethane to give a solution. Thereto, 4-nitrophenyl chloroformate (12.9 g, 64.29 mmol) and pyridine (5.0 ml, 64.29 mmol) were added, followed by stirring at room temperature for 16 hours.

The reaction solution was successively washed with 150 ml of 1N HCl aqueous solution and 150 ml of saturated aqueous sodium chloride solution. Then, the organic phase was dried over anhydrous magnesium sulfate, filtered, and concentrated under reduced pressure. The resulting mixture is was subjected to silica gel column chromatography using methanol and dichloromethane, to give 18.8 g of the target compound as a colorless oil form.

**Example 12-2: Preparation of α-mPEG350-Dab(tBoc)-C)H (compound of formula (23) wherein R is methyl, X is O, n is 7.25 (mean value), m' is 1, and $Z_a$ is a protecting group of tBoc)**

The procedure of Example 6-2 was repeated except that a solution of mPEG350-NPC given in Example 12-1 (1.0 g, 1.94 mmol) dissolved in 20 ml of acetonitrile and 20 ml of distilled water, H-Dab(tBoc)-OH (0.64 g, 2.91 mmol) and diisopropylethylamine (0.50 g, 3.88 mmol) were used and the resulting mixture was subjected to silica gel column chromatography, to give 0.98 g of the target compound.

**Example 12-3: Preparation of α-mPEG350-Dab(tBoc)-DHC (compound of formula (26) wherein R is methyl, X is O, n is 7.25 (mean value), m' is 1, and $Z_a$ is a protecting group of tBoc)**

The procedure of Example 6-3 was repeated except that 7-dehydrocholesterol of formula (4) (582.6 mg, 0.098 mmol) and α-mPEG350-Dab(tBoc)-OH given in Example 12-2 (900 mg, 1.51 mmol) were dissolved in 10 ml of dichloromethane, to which 4-dimethylaminopyridine (208.2 mg, 1.70 mmol) was added, and N-(3-dimethylaminopropyl)-N'-ethyl-carbodiimide (435.5 mg, 2.27 mmol) was used, then the resulting mixture was subjected to silica gel column chromatography, to give 1.23 g of the target compound.

**Example 12-4: Preparation of α-mPEG350-Dab-DHC (compound of formula (27) wherein R is methyl, X is O, n is 7.25 (mean value), and m'is 1)**

The procedure of Example 9-2 was repeated except that a solution of α-mPEG350-Dab(tBoc)-DHC given in Example 12-3 (250 mg, 0.26 mmol) dissolved in 2.5 ml of dichloromethane was added with 2.5 ml of trifluoroacetic acid, and the resulting mixture was subjected to silica gel column chromatography, to give 194 mg of the target compound.

**Example 12-5: Preparation of α-mPEG350-γ-DHC-Dab-DHC (compound of formula (2G) wherein R is methyl, n is 7.25 (mean value), X is O, and m and m' are each 1)**

The procedure of Example 7-3 was repeated except that 7-dehydrocholesterol succinate given in Example 1-1 (56.3 mg, 0.12 mmol) and α-mPEG350-Dab-DHC given in Example 12-4 (100 mg, 0.12 mmol) were dissolved in 5 ml of dichloromethane, to which 4-dimethylaminopyridine (21.3 mg, 0.17 mmol) was added, and N-(3-dimethylaminopropyl)-N'-ethyl-carbodiimide (44.5 mg, 0.23 mmol) was used, to give 141 mg of the target compound.
$^1H$ NMR 400 MHz($CDCl_3$) δ 6.85(bs, 1H), 5.74(m, 1H), 5.59(d, 2H), 5.44(m, 2H), 4.35(t, 2H), 4.33(m, 2H), 4.22(m, 1H), 3.84-3.66(PEG back-bone), 3.39(s, 3H), 2.72(dt, 2H), 2.64(dt, 2H), 2.53-2.39(m, 6H), 2.23-1.51(m, 14H), 1.41-1.06(m, 22H), 0.97(s, 12H), 0.88(d, 6H), 0.87(d, 6H), 0.63(s, 6H)

Table 1 shows the chemical structure of the pegylated 7-dehydrocholesterol derivatives according to the present invention, which are obtained in Examples 1 to 12.

Table 1

| | Compound | Structure | Note |
|---|---|---|---|
| Example 1 | mPEG350-S-DHC ester | 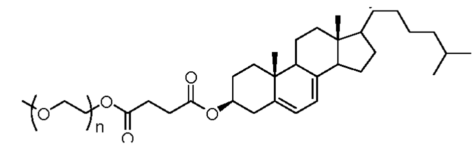 | n = 7.25 (mean value)<br>MW: 816.70 |
| Example 2 | mPEG2K-S-DHC ester | 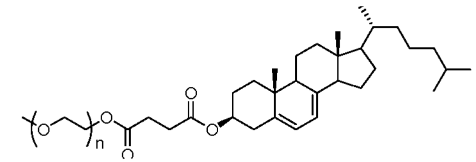 | n = 44.3 (mean value)<br>MW: 2466.70 |
| Example 3 | mTrEG-S-DHC ester | 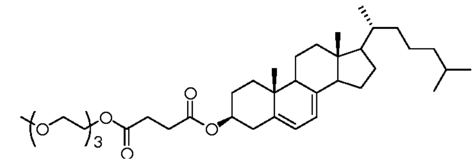 | MW: 630.89 |
| Example 4 | mPEG350-S-DHC amide | 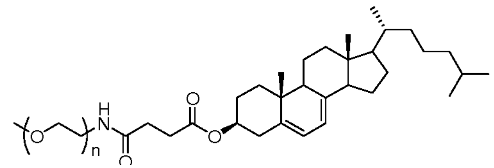 | n = 7.25 (mean value)<br>MW: 815.72 |
| Example 5 | mPEG2K-P-DHC | 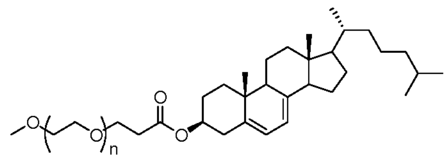 | n = 44.3(mean value)<br>MW: 2438.69 |
| Example 6 | mPEG2K-O-A-DHC | 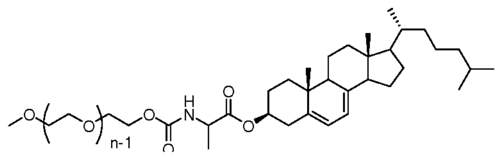 | n = 44.3 (mean value)<br>MW: 2437.71 |

(continued)

| | Compound | Structure | Note |
|---|---|---|---|
| **Example 7** | **mPEG2K-NH-A-DHC** | 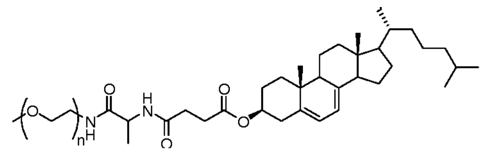 | n = 44.3<br>MW: 2536.79 |
| **Example 8** | **mPEG2K-P-A-DHC** | 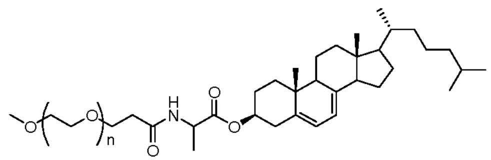 | n = 44.3<br>MW: 2509.78 |
| **Example 9** | **α-mPEG2K-NH-γ-mPEG2K-P-Dab-DHC** | 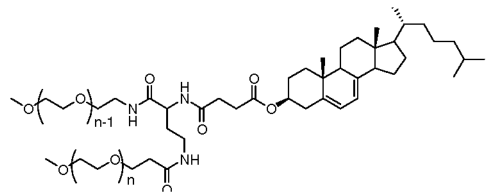 | n = 44.3<br>MW: 4619.89 |
| **Example 10** | **α-mPEG2K-γ-mPEG2K-P-Dab-DHC** | 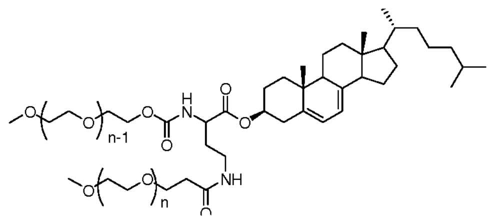 | n = 44.3 (mean value)<br>MW: 4564.82 |
| **Example 11** | **α-mPEG5K-γ-mPEG5K-S-Dab-DHC** | 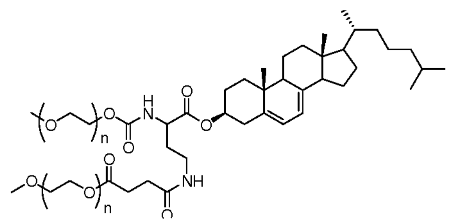 | n = 113.0 (mean value)<br>MW:10564.82 |

(continued)

| | Compound | Structure | Note |
|---|---|---|---|
| **Example 12** | **α-mPEG350-γ-DHC-Dab-DHC** | 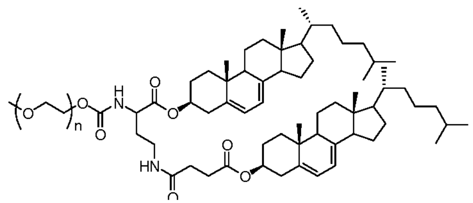 | n = 7.25 (mean value)<br>MW: 1327.44 |

**Experimental Example 1: Solubility Test**

Each of compounds of Examples 1 and 5 and 7-dehydrocholesterol as a comparative material was dissolved in an amount of 100 mg in 100 ml of distilled water, followed by stirring at room temperature for 1 hour. The procedure was repeated until each compound was saturated and those being not dissolved were filtered, to prepare each saturated aqueous solution.

Then, the saturated aqueous solution was put into a weighed empty container (A), followed by weighing (B). Then, the saturated aqueous solution in the container was concentrated in reduced pressure and dried under vacuum, followed by weighing (C). The solubility of each solution was calculated according to the following Equation 1, and the results thereof are shown in Table 2.

[Equation 1]

$$\text{Solubility (S)} = \frac{C - A}{B - C} \times 100$$

**Table 2**

| | 7-Dehydrocholesterol | Compound of Example 1 | Compound of Example 5 |
|---|---|---|---|
| Solubility | Insoluble | 0.02889 (0.289 mg/ml) | 22.783 (227.8 mg/ml) |

From Table 2, it was confirmed that pegylated 7-dehydrocholesterol derivatives of Examples 1 and 5 have surprisingly excellent solubility in water as compared with 7-dehydrocholesterol.

**Experimental Example 2: Thermal Stability Test**

The compound of Example 1 was dissolved in dimethyl sulfoxide at a concentration of 10 mM to prepare a solution. As a comparative material, 7-dehydrocholesterol was dissolved in a mixture of dimethyl sulfoxide and methanol (9:1) at a concentration of 10 mM to prepare another solution. The prepared solutions were each divided in an amount of 1.5 ml, and stored at a constant temperature of 40 °C and 70 °C, with blocking light. After a certain time, each test solution was diluted with methanol to prepare a sample of 1mM. The sample was analyzed with high performance liquid chromatography (HPLC) under the following conditions. The results are shown in Figs. 1 and 2.

Detector: Agilent HPLC 1260
Column: C18 (150 × 4.6 mm/5 um)
Eluent: acetonitrile: methanol= 6: 4
Temperature of Column: 30 °C
Resolution (%) = $[Ct_x/Ct_{0]} \times 100(\%)$
Cto = Peak area for the solution of Example 1at the time of 0
$Ct_x$ = Peak area for the solution of Example 1at the time of X

From Figs. 1 and 2, it was confirmed that the pegylated 7-dehydrocholesterol derivative of Example 1 has superior thermal stability as compared with 7-dehydrocholesterol.

**Experimental Example 3: Photostability Test**

The compound of Example 1 was dissolved in dimethyl sulfoxide at a concentration of 10 mM to prepare a solution. As a comparative material, 7-dehydrocholesterol was dissolved in a mixture of dimethyl sulfoxide and methanol (9:1) at a concentration of 10 mM to prepare another solution. The prepared solutions were each divided in plate wells in an amount of 150 μl, and exposed to UV rays (middle wave, 315 nm, width: 12 cm). After a certain time, each test solution was diluted with methanol to prepare a sample of 1mM. The sample was analyzed with high performance liquid chromatography (HPLC) under the following conditions. The results are shown in Fig. 3.

Detector: Agilent HPLC 1260
Column: C18 (150 × 4.6 mm/5 um)

Eluent : acetonitrile: methanol= 6: 4
Temperature of Column: 30 °C
Resolution (%) = $[Ct_x/Ct_{0]} \times 100(\%)$
$Ct_0$ = Peak area for the solution of Example 1at the time of 0
$Ct_x$ = Peak area for the solution of Example lat the time of X

From Fig. 3, it was confirmed that the pegylated 7-dehydrocholesterol derivative of Example 1 has superior photostability as compared with 7-dehydrocholesterol.

## Claims

1. A pegylated 7-dehydrocholesterol derivative of formula (I):

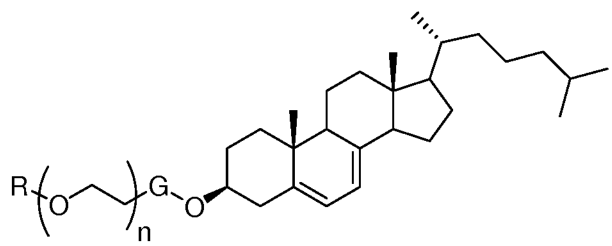

(I)

wherein,

R is hydrogen or $C_1$-$C_6$ alkyl,
G is $-XCOCH_2(CH_2)_mCO-$, $-XCH_2(CH_2)_mCO-$, -XCONHCYCO-,$-XCOCYNHCO(CH_2)_mCH_2CO-$ or $-XCH_2(CH_2)_mCONHCYCO-$,
X is O, NH or S,
Y is hydrogen, $C_1$-$C_6$ alkyl, $(CH_2)_{m'}CH_2NHCO(CH_2)_mCH_2(OCH_2CH_2)_nOR$, $(CH_2)m'CH_2NHCOCH_2(CH_2)_mCO(OCH_2CH_2)_nOR$ or $(CH_2)m'CH_2NHCOCH_2(CH_2)_mCO$-DHC,
n is an integer of 1 to 700, and
m and m' are each independently an integer of 1 to 3.

2. The pegylated 7-dehydrocholesterol derivative of claim 1, which is a compound of formula (1A):

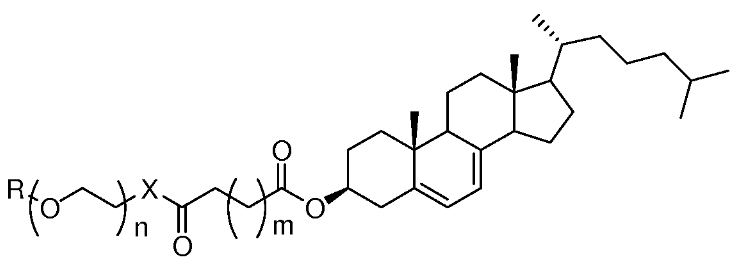

(1A)

wherein,

R is hydrogen or $C_1$-$C_6$ alkyl,
X is O or NH,
n is an integer of 1 to 700, and
m is an integer of 1 to 3.

3. The pegylated 7-dehydrocholesterol derivative of claim 1, which is a compound of formula (1B):

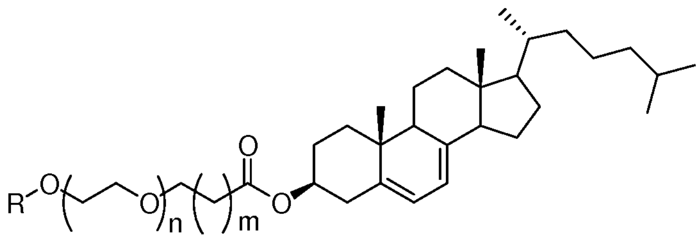

(1B)

wherein,

R is hydrogen or $C_1$-$C_6$ alkyl,
n is an integer of 1 to 700, and
m is an integer of 1 to 3.

**4.** The pegylated 7-dehydrocholesterol derivative of claim 1, which is a compound of formula (2A):

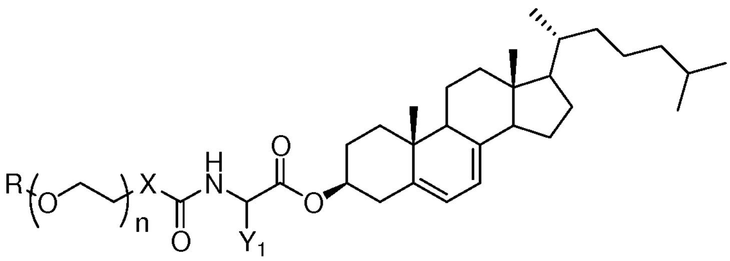

(2A)

wherein,

R is hydrogen or $C_1$-$C_6$ alkyl,
X is O or NH,
n is an integer of 1 to 700, and
$Y_1$ is $C_1$-$C_6$ alkyl.

**5.** The pegylated 7-dehydrocholesterol derivative of claim 1, which is a compound of formula (2B):

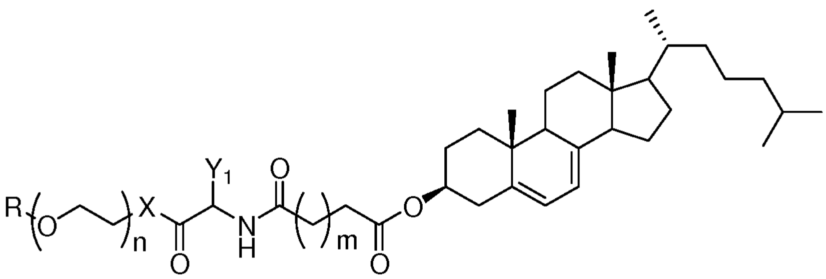

(2B)

wherein,

R is hydrogen or $C_1$-$C_6$ alkyl,
X is O or NH,
n is an integer of 1 to 700,
m is an integer of 1 to 3, and
$Y_1$ is $C_1$-$C_6$ alkyl.

**6.** The pegylated 7-dehydrocholesterol derivative of claim 1, which is a compound of formula (2C):

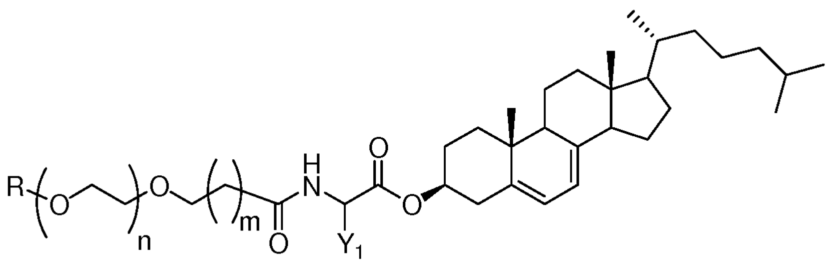

(2C)

wherein,

R is hydrogen or $C_1$-$C_6$ alkyl,
n is an integer of 1 to 700,
m is an integer of 1 to 3, and
$Y_1$ is $C_1$-$C_6$ alkyl.

**7.** The pegylated 7-dehydrocholesterol derivative of claim 1, which is a compound of formula (2D):

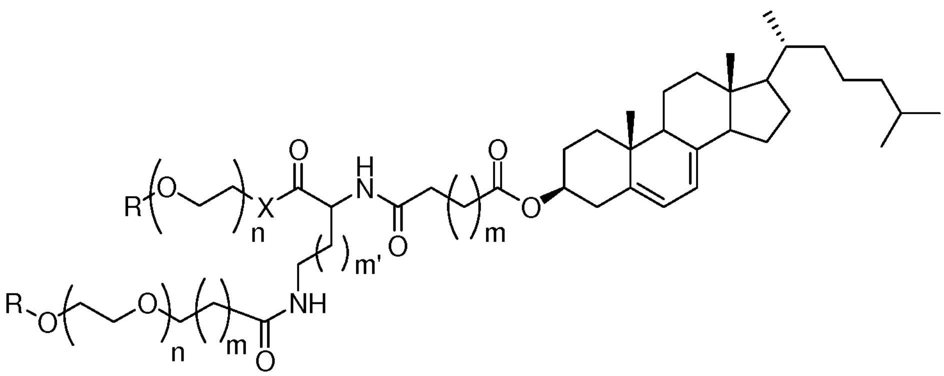

(2D)

wherein,

R is hydrogen or $C_1$-$C_6$ alkyl,
X is O or NH,
n is an integer of 1 to 700, and
m and m' are each independently an integer of 1 to 3.

**8.** The pegylated 7-dehydrocholesterol derivative of claim 1, which is a compound of formula (2E):

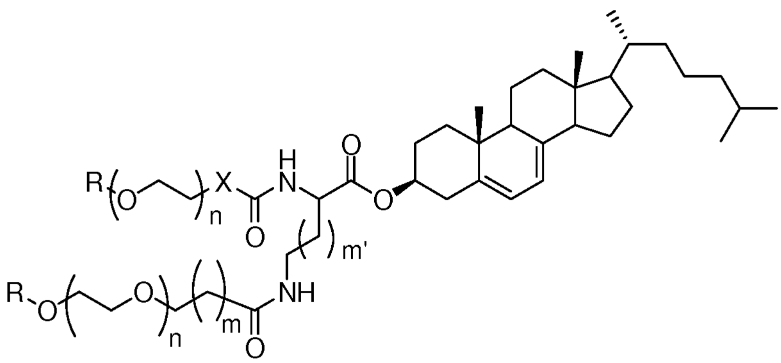

(2E)

wherein,

R is hydrogen or $C_1$-$C_6$ alkyl,

X is O or NH,
n is an integer of 1 to 700,
m and m' are each independently an integer of 1 to 3.

**9.** The pegylated 7-dehydrocholesterol derivative of claim 1, which is a compound of formula (2F):

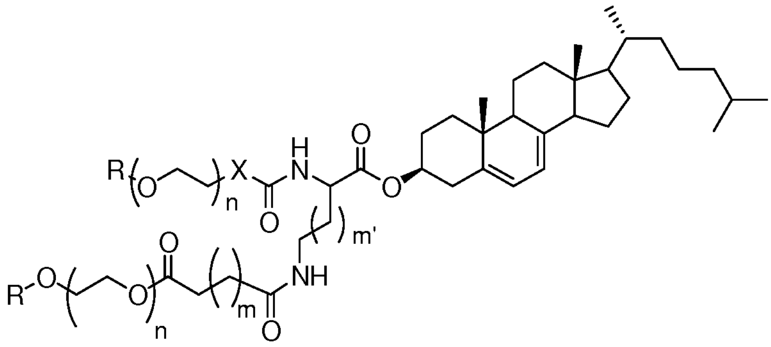

(2F)

wherein,

R is hydrogen or $C_1$-$C_6$ alkyl,
X is O or NH,
n is an integer of 1 to 700, and
m and m' are each independently an integer of 1 to 3.

**10.** The pegylated 7-dehydrocholesterol derivative of claim 1, which is a compound of formula (2G):

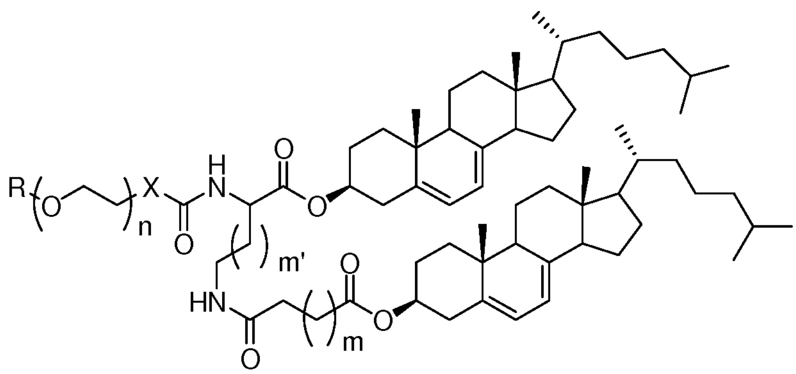

(2G)

wherein,

R is hydrogen or $C_1$-$C_6$ alkyl,
X is O or NH,
n is an integer of 1 to 700, and
m and rri are each independently an integer of 1 to 3.

**11.** The pegylated 7-dehydrocholesterol derivative of any one of claims 1 to 10, wherein n is an integer of 3 to 200.

**12.** The pegylated 7-dehydrocholesterol derivative of any one of claims 1 and 7 to 10, wherein m and m' are 1.

**13.** The pegylated 7-dehydrocholesterol derivative of any one of claims 2, 3, 5 and 6, wherein m is 1.

**14.** A cosmetic composition for wrinkle alleviation and anti-aging, comprising the pegylated 7-dehydrocholesterol derivative of any one of claims 1 to 10.

**15.** A pharmaceutical composition for wrinkle alleviation and anti-aging, comprising the pegylated 7-dehydrocholesterol

derivative of any one of claims 1 to 10.

**16.** A functional food for wrinkle alleviation and anti-aging, comprising the pegylated 7-dehydrocholesterol derivative of any one of claims 1 to 10.

【FIGURE 1】

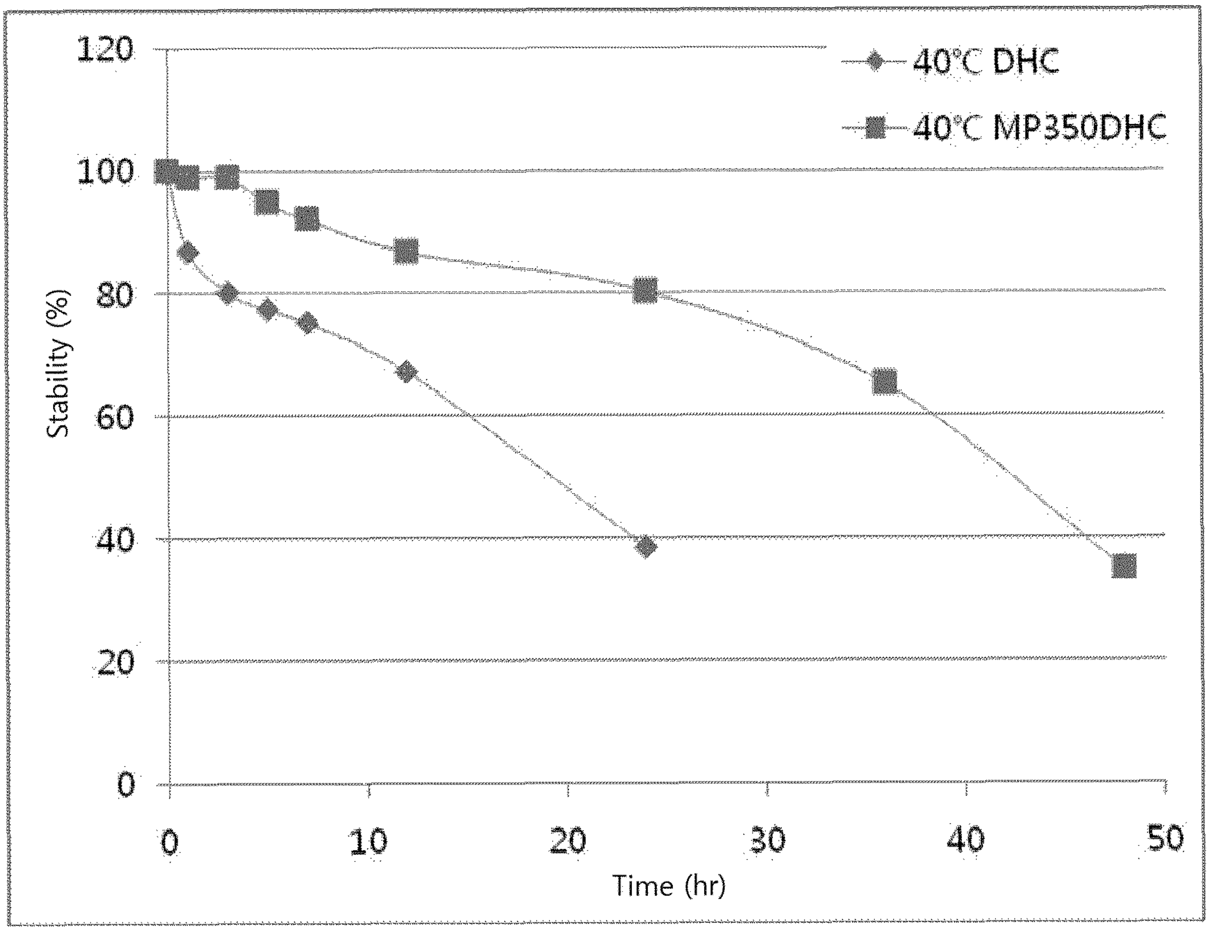
40°C DHC
40°C MP350DHC
Stability (%)
Time (hr)
120
100
80
60
40
20
0
0
10
20
30
40
50

【FIGURE 2】

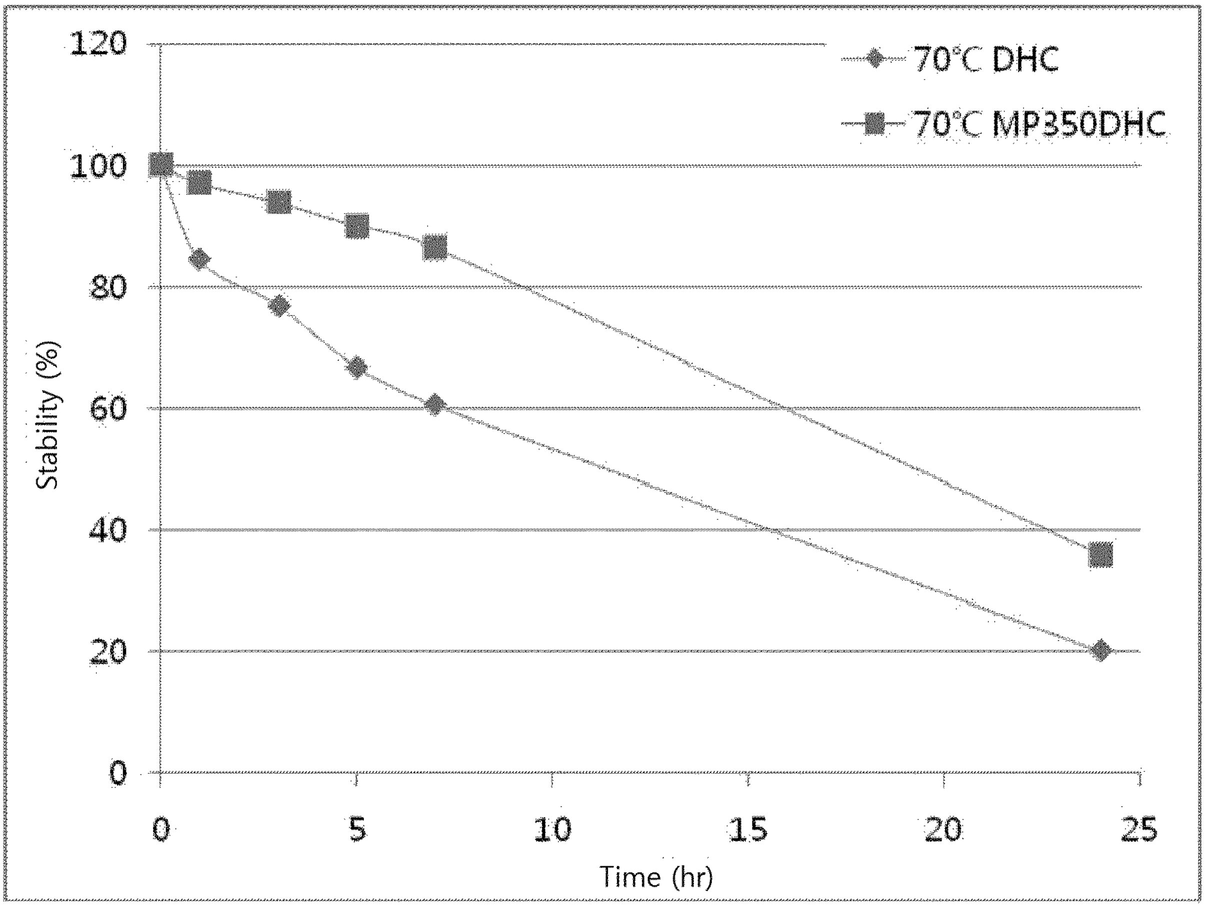
120
100
80
60
40
20
0
Stability (%)
0
5
10
15
20
25
Time (hr)
70℃ DHC
70℃ MP350DHC

【FIGURE 3】

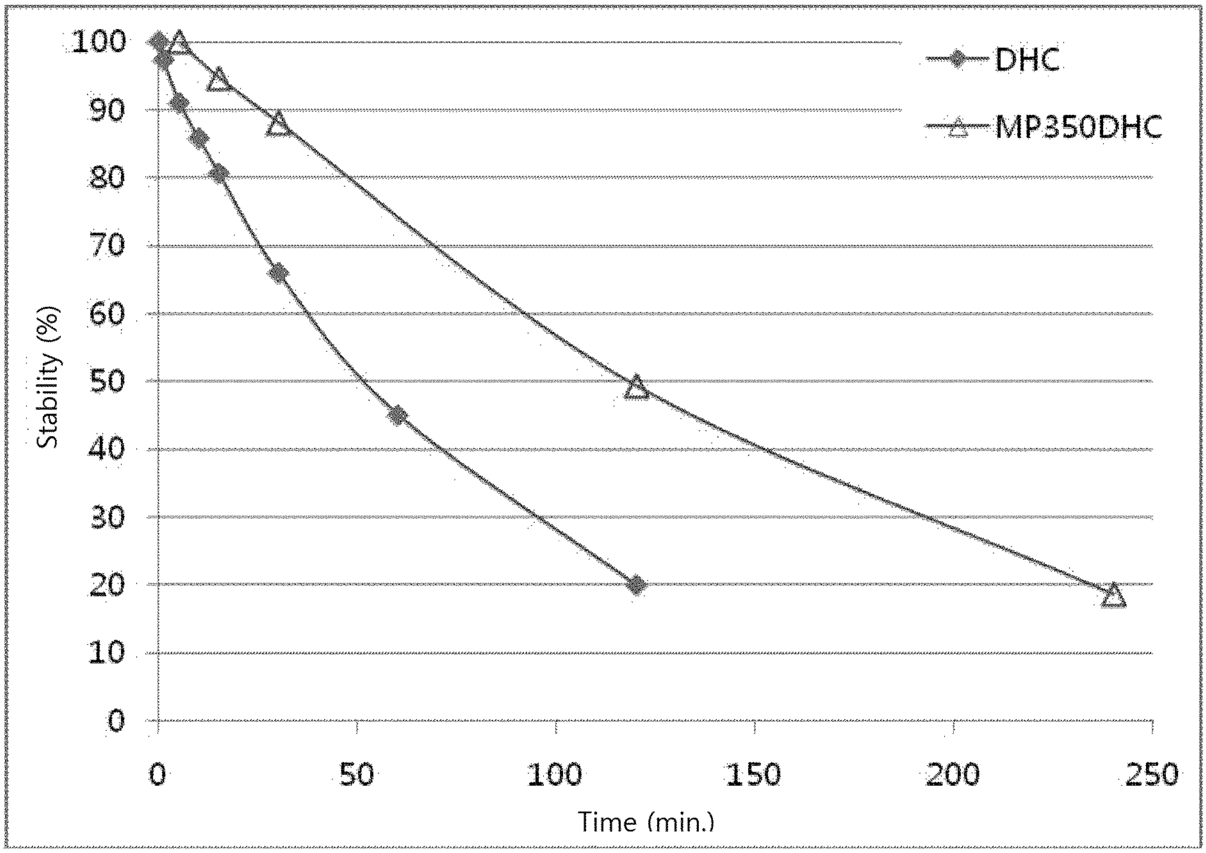

DHC
MP350DHC
Stability (%)
Time (min.)
100
90
80
70
60
50
40
30
20
10
0
0
50
100
150
200
250

**INTERNATIONAL SEARCH REPORT**

International application No.
**PCT/KR2015/000910**

A. CLASSIFICATION OF SUBJECT MATTER

***C07J 9/00(2006.01)i***

According to International Patent Classification (IPC) or to both national classification and IPC

B. FIELDS SEARCHED

Minimum documentation searched (classification system followed by classification symbols)

C07J 9/00; A61K 31/70; A61K 47/32; C07C 401/00; A61K 31/675; A61K 31/683; A61K 47/48; A61P 3/04; A61K 9/127; C07J 43/00

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: PEGylated 7-dehydrocholesterol(PEGylated 7-dehydrocholesterol), anti-wrinkle, anti aging

C. DOCUMENTS CONSIDERED TO BE RELEVANT

| Category* | Citation of document, with indication, where appropriate, of the relevant passages | Relevant to claim No. |
|---|---|---|
| X<br>Y<br>A | US 2001-0025058 A1 (BOROWY-BOROWSKI, H. et al.) 27 September 2001<br>See abstract; and claims 39, 41. | 1-2,11-13<br>14-16<br>3-10 |
| Y | US 2010-0036140 A1 (ZHANG, W.) 11 February 2010<br>See paragraphs [0058]-[0059]; and claims 1, 10. | 14-16 |
| A | US 2012-0010173 A1 (GONNISSEN, Y. R. J. P. et al. ) 12 January 2012<br>See paragraph [0148]. | 1-16 |
| A | US 2008-0317767 A1 (BRAXMEIER, Tobias et al.) 25 December 2008<br>US 2008-0317767 A1 (BRAXMEIER, T. et al.) 25 December 2008<br>See claims 1, 21. | 1-16 |
| A | US 2004-0120997 A1 (PANZNER, S. et al.) 24 June 2004<br>See abstract; and claim 1. | 1-16 |

☐ Further documents are listed in the continuation of Box C. ☒ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 15 MAY 2015 (15.05.2015) | **15 MAY 2015 (15.05.2015)** |

| Name and mailing address of the ISA/**KR** | Authorized officer |
|---|---|
| Korean Intellectual Property Office<br>Government Complex-Daejeon, 189 Seonsa-ro, Daejeon 302-701, Republic of Korea<br>Facsimile No. 82-42-472-7140 | Telephone No. |

Form PCT/ISA/210 (second sheet) (July 2009)

INTERNATIONAL SEARCH REPORT
Information on patent family members

International application No.
PCT/KR2015/000910

| Patent document cited in search report | Publication date | Patent family member | Publication date |
|---|---|---|---|
| US 2001-0025058 A1 | 27/09/2001 | AU 2000-22734 A1 | 14/11/2000 |
| | | AU 2000-22734 B2 | 15/07/2004 |
| | | CA 2369300 A1 | 19/10/2000 |
| | | CA 2369300 C | 08/06/2010 |
| | | CA 2436374 A1 | 15/08/2002 |
| | | CN 1352568 A | 05/06/2002 |
| | | EP 1159006 A2 | 05/12/2001 |
| | | EP 1159006 B1 | 10/09/2008 |
| | | EP 1363671 A2 | 26/11/2003 |
| | | EP 1810694 A2 | 25/07/2007 |
| | | EP 1810694 A3 | 09/01/2008 |
| | | EP 1985308 A2 | 29/10/2008 |
| | | EP 1985308 A3 | 16/11/2011 |
| | | JP 2002-541216 A | 03/12/2002 |
| | | JP 2004-521900 A | 22/07/2004 |
| | | KR 10-2002-0012167 A | 15/02/2002 |
| | | US 06045826 A | 04/04/2000 |
| | | US 2004-0156871 A1 | 12/08/2004 |
| | | US 2008-0070981 A1 | 20/03/2008 |
| | | US 2009-0258038 A9 | 15/10/2009 |
| | | US 2010-0063140 A1 | 11/03/2010 |
| | | US 6191172 B1 | 20/02/2001 |
| | | US 6632443 B2 | 14/10/2003 |
| | | US 7645816 B2 | 12/01/2010 |
| | | US 8524696 B2 | 03/09/2013 |
| | | WO 00-61189 A2 | 19/10/2000 |
| | | WO 00-61189 A3 | 11/01/2001 |
| | | WO 02-062392 A2 | 15/08/2002 |
| | | WO 02-062392 A3 | 14/11/2002 |
| US 2010-0036140 A1 | 11/02/2010 | CN 1962683 A | 16/05/2007 |
| | | GB 0912378 D0 | 26/08/2009 |
| | | GB 2458080 A | 09/09/2009 |
| | | GB 2458080 B | 07/12/2011 |
| | | WO 2008-074216 A1 | 26/06/2008 |
| US 2012-0010173 A1 | 12/01/2012 | AU 2010-225035 A1 | 29/09/2011 |
| | | AU 2010-225035 A1 | 23/09/2010 |
| | | CA 2754168 A1 | 23/09/2010 |
| | | CN 102439018 A | 02/05/2012 |
| | | EP 2408791 A1 | 25/01/2012 |
| | | JP 2012-520840 A | 10/09/2012 |
| | | RU 2011142154 A | 27/04/2013 |
| | | US 2012-0172336 A2 | 05/07/2012 |
| | | WO 2010-105910 A1 | 23/09/2010 |
| US 2008-0317767 A1 | 25/12/2008 | AT 442865 T | 15/10/2009 |
| | | AU 2005-231622 A1 | 20/10/2005 |
| | | AU 231622 B2 | 26/05/2011 |

Form PCT/ISA/210 (patent family annex) (July 2009)

INTERNATIONAL SEARCH REPORT
Information on patent family members

International application No.
PCT/KR2015/000910

| Patent document cited in search report | Publication date | Patent family member | Publication date |
|---|---|---|---|
| | | CA 2562266 A1 | 20/10/2005 |
| | | DE 602005016658 D1 | 29/10/2009 |
| | | EP 1732612 A1 | 20/12/2006 |
| | | EP 1732612 B1 | 16/09/2009 |
| | | EP 2119455 A1 | 18/11/2009 |
| | | WO 2005-097199 A1 | 20/10/2005 |
| US 2004-0120997 A1 | 24/06/2004 | AT 364620 T | 15/07/2007 |
| | | AT 430759 T | 15/05/2009 |
| | | AU 2002-244726 A1 | 04/09/2002 |
| | | AU 302364 B2 | 03/01/2008 |
| | | BR 0207776 A | 23/03/2004 |
| | | CA 2438910 A1 | 29/08/2002 |
| | | CA 2438910 C | 13/10/2009 |
| | | CN 00231493 C | 14/12/2005 |
| | | CN 100492876 A | 28/04/2004 |
| | | DE 10109898 A1 | 05/09/2002 |
| | | DE 50210313 D1 | 26/07/2007 |
| | | DE 50213524 D1 | 18/06/2009 |
| | | EP 1363932 A2 | 26/11/2003 |
| | | EP 1363932 B1 | 13/06/2007 |
| | | EP 1363933 A2 | 26/11/2003 |
| | | EP 1363933 B1 | 06/05/2009 |
| | | EP 1806354 A2 | 11/07/2007 |
| | | JP 04470197 B2 | 02/06/2010 |
| | | JP 2004-521917 A | 22/07/2004 |
| | | JP 2004-525902 A | 26/08/2004 |
| | | US 2004-0131666 A1 | 08/07/2004 |
| | | US 2008-0113017 A1 | 15/05/2008 |
| | | US 7312206 B2 | 25/12/2007 |
| | | US 7407947 B2 | 05/08/2008 |
| | | WO 02-066489 A2 | 29/08/2002 |
| | | WO 02-066489 A3 | 03/01/2003 |
| | | WO 02-066490 A2 | 29/08/2002 |
| | | WO 02-066490 A3 | 24/04/2003 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 20010002281
- US 20130017234 A
- KR 100568600
- US 5342833 A
- US 5747478 A


### Non-patent literature cited in the description


- **J.M. HARRIS.** *Advanced Drug Delivery Reviews,* 2002, vol. 54, 459-476
- **SANDLER ; KARO.** Polymer Synthesis. Academic Press, New York, vol. 3, 138-161